# EUROPEAN PATENT APPLICATION

(11) **EP 2 476 415 A2**
(43) Date of publication of application: **18.07.2012**
(21) Application number: 12161224.6
(22) Date of filing: 12.10.2004
(51) Int. Cl.: A61K 31/167, A61P 19/08, A61P 19/10, A61P 3/00, A61P 5/00, A61K 31/00, A61K 31/165, A61K 31/21, A61K 31/32, A61K 31/405, A61K 31/4704, A61K 31/66

(54) **Treating bone-related disorders with selective androgen receptor modulators**

(30) Priority: 14.10.2003 US 510138 P; 28.09.2004 US 613206 P
(62) Divisional of application: 04809913.9
(71) Applicant: University of Tennessee Research Foundation, Knoxville, TN 37996-4122 (US)
(72) Inventor: Dalton, James, Upper Arlington, OH 43220 (US); Miller, Duane D., Germantown, TN 38139 (US); Steiner, Mitchell S., Germantown, TN 38138 (US); Veverka, Karen A., Cordova, TN 38018 (US); Kearby, Jeffrey, Hilliard, OH 43024 (US)
(74) Representative: Lord, Hilton David

(57) **Abstract**

This invention provides method of treating, preventing, suppressing, inhibiting, or reducing the risk of developing a bone-related disorder, for example osteoporosis, osteopenia, increased bone resorption, bone fracture, bone frailty and/or loss of bone mineral density (BMD), by administering a therapeutically effective amount of a selective androgen receptor modulator (SARM) and/or its analogue, derivative, isomer, metabolite pharmaceutically acceptable salt, pharmaceutical product, hydrate, N-oxide, or any combination thereof. The invention also provides methods of decreasing fat mass (FM) and increasing lean mass, comprising administering same.

## Description

### FIELD OF INVENTION

This invention provides method of treating, preventing, suppressing, inhibiting, or reducing the risk of developing a bone-related disorder, for example osteoporosis, osteopenia, increased bone resorption, bone fracture, bone frailty and/or loss of bone mineral density (BMD), by administering a therapeutically effective amount of a selective androgen receptor modulator (SARM) and/or its analogue, derivative, isomer, metabolite, pharmaceutically acceptable salt, pharmaceutical product, hydrate, N-oxide, or any combination thereof. The invention also provides methods of decreasing fat mass (FM) and increasing lean mass, comprising administering same.

### BACKGROUND OF THE INVENTION

BMD decreases with age in both males and females. Decreased amounts of bone mineral content (BMC) and BMD correlate with decreased bone strength and predispose patients to fracture.

Osteoporosis is a systemic skeletal disease, characterized by low bone mass and deterioration of bone tissue, with a consequent increase in bone fragility and susceptibility to fracture. In the U.S., the condition affects more than 25 million people and causes more than 1.3 million fractures each year, including 500,000 spine, 250,000 hip and 240,000 wrist fractures annually. Hip fractures are the most serious consequence of osteoporosis, with 5-20% of patients dying within one year, and over 50% of survivors being incapacitated. The elderly are at greatest risk of osteoporosis, and the problem is therefore predicted to increase significantly with the aging of the population. Worldwide fracture incidence is forecasted to increase three-fold over the next 60 years, and one study estimated that there will be 4.5 million hip fractures worldwide in 2050.

Given the high incidence of osteoporosis and other bone-related disorders, bone-related disorders are of a major clinical health concern to both males and females. New innovative approaches are urgently needed at both the basic science and clinical levels to decrease the incidence of bone-related disorders.

### SUMMARY OF THE INVENTION

In one embodiment, the present invention provides a method of treating a subject having a bone-related disorder, comprising the step of administering to the subject a SARM compound. In another embodiment, the method comprises administering an analogue, derivative, isomer, metabolite, pharmaceutically acceptable salt, pharmaceutical product, hydrate or N-oxide of the SARM compound, or any combination thereof.

In another embodiment, the present invention provides a method of reducing the incidence of a bone-related disorder in a subject, comprising administering to the subject a SARM compound. In another embodiment, the method comprises administering an analogue, derivative, isomer, metabolite, pharmaceutically acceptable salt, pharmaceutical product, hydrate or N-oxide of the SARM compound, or any combination thereof.

In another embodiment, the present invention provides a method of increasing bone strength of a subject, comprising administering to the subject a SARM compound. In another embodiment, the method comprises administering an analogue, derivative, isomer, metabolite, pharmaceutically acceptable salt, pharmaceutical product, hydrate or N-oxide of the SARM compound, or any combination thereof.

In another embodiment, the present invention provides a method of increasing bone mass of a subject, comprising administering to the subject a SARM compound. In another embodiment, the method comprises administering an analogue, derivative, isomer, metabolite, pharmaceutically acceptable salt, pharmaceutical product, hydrate or N-oxide of the SARM compound, or any combination thereof.

In another embodiment, the present invention provides method of reducing the incidence of a bone resorption in a subject, comprising administering to the subject a SARM compound. In another embodiment, the method comprises administering an analogue, derivative, isomer, metabolite, pharmaceutically acceptable salt, pharmaceutical product, hydrate or N-oxide of the SARM compound, or any combination thereof.

In another embodiment, the present invention provides method of reducing an FM of a subject, comprising administering to the subject a SARM compound. In another embodiment, the method comprises administering an analogue, derivative, isomer, metabolite, pharmaceutically acceptable salt, pharmaceutical product, hydrate or N-oxide of the SARM compound, or any combination thereof.

In another embodiment, the present invention provides method of reducing an incidence of an increase in a fat mass (FM) of a subject, comprising administering to the subject a SARM compound. In another embodiment, the method comprises administering an analogue, derivative, isomer, metabolite, pharmaceutically acceptable salt, pharmaceutical product, hydrate or N-oxide of the SARM compound, or any combination thereof.

In another embodiment, the present invention provides method of increasing a muscle mass in a subject, comprising administering to the subject a SARM compound. In another embodiment, the method comprises administering an analogue, derivative, isomer, metabolite, pharmaceutically acceptable salt, pharmaceutical product, hydrate or N-oxide of the SARM compound, or any combination thereof.

In another embodiment, the present invention provides method of reducing an incidence of a decrease in a muscle mass in a subject, comprising administering to the subject a SARM compound. In another embodiment, the method comprises administering an analogue, derivative, isomer, metabolite, pharmaceutically acceptable salt, pharmaceutical product, hydrate or N-oxide of the SARM compound, or any combination thereof.

In another embodiment, the present invention provides method of increasing a lean mass in a subject, comprising administering to the subject a SARM compound. In another embodiment, the method comprises administering an analogue, derivative, isomer, metabolite, pharmaceutically acceptable salt, pharmaceutical product, hydrate or N-oxide of the SARM compound, or any combination thereof.

### BRIEF DESCRIPTION OF THE DRAWINGS

The present invention will be understood and appreciated more fully from the following detailed description taken in conjunction with the appended drawings in which:

Figure 1. Whole body BMD at day 120. (mean ± standard error of measurement [S.E.M.]). a = P < 0.05 vs. OVX controls; b = P < 0.05 vs. intact controls.

Figure 2. Lumbar vertebrae (L5-L6) BMD at day 120 (mean ± S.E.M). a = P < 0.05 vs. OVX controls; b = P < 0.05 vs. intact controls.

Figure 3. Lumbar vertebrae (L2-L4) BMD at day 120 (mean ± S.E.M).

Figure 4. Femoral region 4 BMD at day 120 (mean ± S.E.M). a = P < 0.05 vs. OVX controls; b = P < 0.05 vs. intact controls.

Figure 5. Proximal femur BMD at day 120 (mean ± S.E.M).

Figure 6. Cortical thickness of the mid-shaft femur at day 120 (mean ± S.E.M). a = P < 0.05 vs. OVX controls; b = P < 0.05 vs. intact controls.

Figure 7. Cortical content of the mid-shaft femur at day 120 (mean ± S.E.M). a = P < 0.05 vs. OVX controls; b = P < 0.05 vs. intact controls.

Figure 8. Periosteal circumference of the mid-shaft femur at day 120 (mean ± S.E.M). a = P < 0.05 vs. OVX controls; b = P < 0.05 vs. intact controls.

Figure 9. Trabecular density of the distal femur at day 120 (mean ± S.E.M). a = P < 0.05 vs. OVX controls; b = P < 0.05 vs. intact controls.

Figure 10. Femoral maximum load at day 120 (mean ± S.E.M). a = P < 0.05 vs. OVX controls; b = P < 0.05 vs. intact controls.

Figure 11. Compression strength of the L5 vertebra at day 120 (mean ± S.E.M).

Figure 12. (A) Percent change in BMC at day 120. (B) time course of change in BMC. Data are presented as mean ± S.E.M.

Figure 13. Percent change in BMC at day 30 (mean ± S.E.M).

Figure 14. Body weight at day 120 (mean ± S.E.M). a = P < 0.05 vs. OVX controls; b = P < 0.05 vs. intact controls.

Figure 15. Percent FM at day 120 (mean ± S.E.M). a = P < 0.05 vs. OVX controls; b = P < 0.05 vs. intact controls.

Figure 16. Serum levels of osteocalcin at day 120 (mean ± S.E.M). a = P < 0.05 vs. OVX controls; b = P < 0.05 vs. intact controls.

Figure 17. Whole body BMC at day 210 (mean ± S.E.M). a = P < 0.05 vs. OVX controls; b = P < 0.05 vs. intact controls.

Figure 18. Lumbar vertebrae at day 210 (L5-L6) BMC (mean ± S.E.M). a = P < 0.05 vs. OVX controls; b = P < 0.05 vs. intact controls. Intact controls were sacrificed at day 210.

Figure 19. Femoral region 4 BMD at day 210 (mean ± S.E.M). a = P < 0.05 vs. OVX controls; b = P < 0.05 vs. intact controls. Intact controls were sacrificed at day 210.

Figure 20. Cortical content of the mid-shaft femur at day 210 (mean ± S.E.M). a = P < 0.05 vs. OVX controls; b = P < 0.05 vs. intact controls.

Figure 21. Cortical thickness of the mid-shaft femur at day 210 (mean ± S.E.M). a = P < 0.05 vs. OVX controls; b = P < 0.05 vs. intact controls.

Figure 22. Periosteal circumference of the mid-shaft femur at day 210 (mean ± S.E.M). a = P < 0.05 vs. OVX controls; b = P < 0.05 vs. intact controls.

Figure 23. Trabecular density of the distal femur at day 210 (mean ± S.E.M). a = P < 0.05 vs. OVX controls; b = P < 0.05 vs. intact controls.

Figure 24. Femoral maximum load determined by 3-point bending at day 210 (mean ± S.E.M). a = P < 0.05 vs. OVX controls; b = P < 0.05 vs. intact controls.

Figure 25. Body weight at day 210 (mean ± S.E.M). a = P < 0.05 vs. OVX controls; b = P < 0.05 vs. intact controls.

Figure 26. Percent FM at day 210 (mean ± S.E.M). a = P < 0.05 vs. OVX controls; b = P < 0.05 vs. intact controls.

Figure 27. Whole body BMD at day 120. a = P < 0.05 vs. OVX controls; b = P < 0.05 vs. intact controls.

Figure 28. BMD of L5-L6 vertebrae at day 120. a = P < 0.05 vs. OVX controls; b = P < 0.05 vs. intact controls.

Figure 29. Whole body BMD at day 210. a = P < 0.05 vs. OVX controls; b = P < 0.05 vs. intact controls.

Figure 30. BMD of L5-L6 vertebrae at day 210. a = P < 0.05 vs. OVX controls; b = P < 0.05 vs. intact controls.

Figure 31. Body weight at day 120. a = P < 0.05 vs. OVX controls; b = P < 0.05 vs. intact controls.

Figure 32. Body weight at day 210. a = P < 0.05 vs. OVX controls; b = P < 0.05 vs. intact controls.

Figure 33. Percent FM at day 120. a = P < 0.05 vs. OVX controls; b = P < 0.05 vs. intact controls.

Figure 34. Percent FM at day 210. a = P < 0.05 vs. OVX controls; b = P < 0.05 vs. intact controls.

### DETAILED DESCRIPTION OF THE INVENTION

The present invention provides methods of treating, preventing, suppressing, inhibiting or reducing the incidence of a bone-related disorder in a subject, by administering to the subject a selective androgen receptor modulator (SARM) compound and/or its analogue, derivative, isomer, metabolite, pharmaceutically acceptable salt, pharmaceutical product, hydrate, N-oxide, or any combination thereof. The present invention further provides methods of increasing a bone strength or bone mass of a subject, increasing a muscle mass of a subject, and decreasing an FM of a subject, by administering same.

In another embodiment, the present invention provides a method of reducing the incidence of a bone-related disorder in a subject, comprising administering to the subject a SARM compound. In another embodiment, the method comprises administering an analogue, derivative, isomer, metabolite, pharmaceutically acceptable salt, pharmaceutical product, hydrate or N-oxide of the SARM compound, or any combination thereof.

In another embodiment, the present invention provides a method of preventing a bone-related disorder in a subject, comprising administering one of the above compounds. In another embodiment, the present invention provides a method of suppressing a bone-related disorder in a subject, comprising administering same. In another embodiment, the present invention provides a method of inhibiting a bone-related disorder in a subject, comprising administering same.

In one embodiment, the bone-related disorder is osteoporosis. In another embodiment, the bone-related disorder is osteopenia. In another embodiment, the bone-related disorder is increased bone resorption. In another embodiment, the bone-related disorder is bone fracture. In another embodiment, the bone-related disorder is bone frailty. In another embodiment, the bone-related disorder is a loss of BMD. In another embodiment, the bone-related disorder is any combination of osteoporosis, osteopenia, increased bone resorption, bone fracture, bone frailty and loss of BMD. Each disorder represents a separate embodiment of the present invention.

"Osteoporosis" refers, in one embodiment, to a thinning of the bones with reduction in bone mass due to depletion of calcium and bone protein. In another embodiment, osteoporosis is a systemic skeletal disease, characterized by low bone mass and deterioration of bone tissue, with a consequent increase in bone fragility and susceptibility to fracture. In osteoporotic patients, bone strength is abnormal, in one embodiment, with a resulting increase in the risk of fracture. In another embodiment, osteoporosis depletes both the calcium and the protein collagen normally found in the bone, in one embodiment, resulting in either abnormal bone quality or decreased bone density. In another embodiment, bones that are affected by osteoporosis can fracture with only a minor fall or injury that normally would not cause a bone fracture. The fracture can be, in one embodiment, either in the form of cracking (as in a hip fracture) or collapsing (as in a compression fracture of the spine). The spine, hips, and wrists are common areas of osteoporosis-induced bone fractures, although fractures can also occur in other skeletal areas. Unchecked osteoporosis can lead, in another embodiment, to changes in posture, physical abnormality, and decreased mobility.

In one embodiment, the osteoporosis results from androgen deprivation. In another embodiment, the osteoporosis follows androgen deprivation. In another embodiment, the osteoporosis is primary osteoporosis. In another embodiment, the osteoporosis is secondary osteoporosis. In another embodiment, the osteoporosis is postmenopausal osteoporosis. In another embodiment, the osteoporosis is juvenile osteoporosis. In another embodiment, the osteoporosis is idiopathic osteoporosis. In another embodiment, the osteoporosis is senile osteoporosis.

In another embodiment, the primary osteoporosis is Type I primary osteoporosis. In another embodiment, the primary osteoporosis is Type II primary osteoporosis. Each type of osteoporosis represents a separate embodiment of the present invention.

Osteoporosis and osteopenia are, in another embodiment, systemic skeletal diseases characterized by low bone mass and microarchitectural deterioration of bone tissue. "Microarchitectural deterioration" refers, in one embodiment, to thinning of the trabeculae (defined below) and the loss of inter-trabecular connections in bone. In another embodiment, "osteoporosis" is defined as having a BMD 2.5 standard deviations (SD) or more below the young adult mean. In another embodiment, "osteoporosis" is defined as having a BMC 2.5 SD or more below the young adult mean. In another embodiment, "osteoporosis" is defined as having a BMD 2.0 SD or more below the young adult mean. In another embodiment, "osteoporosis" is defined as having a BMC 2.0 SD or more below the young adult mean. In another embodiment, "osteoporosis" is defined as having a BMD 3.0 SD or more below the young adult mean. In another embodiment, "osteoporosis" is defined as having a BMC 3.0 SD or more below the young adult mean. Each definition of osteoporosis or osteopenia represents a separate embodiment of the present invention.

In another embodiment, "osteoporosis" is defined as having a BMD 2.5 SD below the young adult mean. In another embodiment, "osteoporosis" is defined as having a BMC 2.5 SD below the young adult mean. In another embodiment, "osteoporosis" is defined as having a BMD 2.0 SD below the young adult mean. In another embodiment, "osteoporosis" is defined as having a BMC 2.0 SD below the young adult mean. In another embodiment, "osteoporosis" is defined as having a BMD 3.0 SD below the young adult mean. In another embodiment, "osteoporosis" is defined as having a BMC 3.0 SD below the young adult mean. Each definition of osteoporosis represents a separate embodiment of the present invention.

Methods for assessing osteoporosis and osteopenia are well known in the art. For example, in one embodiment, a patient's BMD, measured by densitometry and expressed in g/cm², is compared with a "normal value," which is the mean BMD of sex-matched young adults at their peak bone mass, yielding a "T score." In another embodiment, Z-score, the amount of bone loss in a patient is compared with the expected loss for individuals of the same age and sex. In another embodiment, "osteoporosis" is defined as having a T score 2.5 SD or more below the young adult mean. In another embodiment, "osteoporosis" is defined as having a Z score 2.5 SD or more below the young adult mean. In another embodiment, "osteoporosis" is defined as having a T score 2.0 SD or more below the young adult mean. In another embodiment, "osteoporosis" is defined as having a Z score 2.0 SD or more below the young adult mean. In another embodiment, "osteoporosis" is defined as having a T score 3.0 SD or more below the young adult mean. In another embodiment, "osteoporosis" is defined as having a Z score 3.0 SD or more below the young adult mean.

In another embodiment, "osteoporosis" is defined as having a T score 2.5 SD below the young adult mean. In another embodiment, "osteoporosis" is defined as having a Z score 2.5 SD below the young adult mean. In another embodiment, "osteoporosis" is defined as having a T score 2.0 SD below the young adult mean. In another embodiment, "osteoporosis" is defined as having a Z score 2.0 SD below the young adult mean. In another embodiment, "osteoporosis" is defined as having a T score 3.0 SD below the young adult mean. In another embodiment, "osteoporosis" is defined as having a Z score 3.0 SD below the young adult mean. Each definition of osteoporosis represents a separate embodiment of the present invention.

The term "BMD" is, in one embodiment, a measured calculation of the true mass of bone. The absolute amount of bone as measured by BMD generally correlates with bone strength and its ability to bear weight. By measuring BMD, it is possible to predict fracture risk in the same manner that measuring blood pressure can help predict the risk of stroke.

BMD, in one embodiment, can be measured by known BMD mapping techniques. In one embodiment, bone density of the hip, spine, wrist, or calcaneus may be measured by a variety of techniques. The preferred method of BMD measurement is dual-energy x-ray densitometry (DEXA). BMD of the hip, antero-posterior (AP) spine, lateral spine, and wrist can be measured using this technology. Measurement at any site predicts overall risk of fracture, but information from a specific site is the best predictor of fracture at that site. Quantitative computerized tomography (QCT) is also used to measure BMD of the spine. See for example, "Nuclear Medicine: "Quantitative Procedures" by Wahner H W, et al, published by Toronto Little, Brown & Co., 1983, pages 107-132; "Assessment of Bone Mineral Part 1," J Nucl Medicine, pp 1134-1141 (1984); and "Bone Mineral Density of The Radius" J Nucl Medicine 26: 13-39 (1985). Each method of measuring BMD represents a separate embodiment of the present invention.

"Osteopenia" refers, in one embodiment, to having a BMD or BMC between 1 and 2.5 SD below the young adult mean. In another embodiment, "osteopenia" refers to decreased calcification or density of bone. This term encompasses, in one embodiment, all skeletal systems in which such a condition is noted. Each definition or means of diagnosis of the disorders disclosed in the present invention represents a separate embodiment of the present invention.

In one embodiment, the term "bone fracture" refers to a breaking of bones, and encompasses both vertebral and non-vertebral bone fractures. The term "bone frailty" refers, in one embodiment, to a weakened state of the bones that predisposes them to fractures.

In one embodiment, the osteoporosis, osteopenia, increased bone resorption, bone fracture, bone frailty, loss of BMD, and other diseases or disorders of the present invention are caused by a hormonal disorder, disruption or imbalance. In another embodiment, these conditions occur independently of a hormonal disorder, disruption or imbalance. Each possibility represents a separate embodiment of the present invention.

In one embodiment, the hormonal disorder, disruption or imbalance comprises an excess of a hormone. In another embodiment, the hormonal disorder, disruption or imbalance comprises a deficiency of a hormone. In one embodiment, the hormone is a steroid hormone. In another embodiment, the hormone is an estrogen. In another embodiment, the hormone is an androgen. In another embodiment, the hormone is a glucocorticoid. In another embodiment, the hormone is a cortico-steroid. In another embodiment, the hormone is Luteinizing Hormone (LH). In another embodiment, the hormone is Follicle Stimulating Hormone (FSH). In another embodiment, the hormone is any other hormone known in the art. In another embodiment, the hormonal disorder, disruption or imbalance is associated with menopause. Each possibility represents a separate embodiment of the present invention.

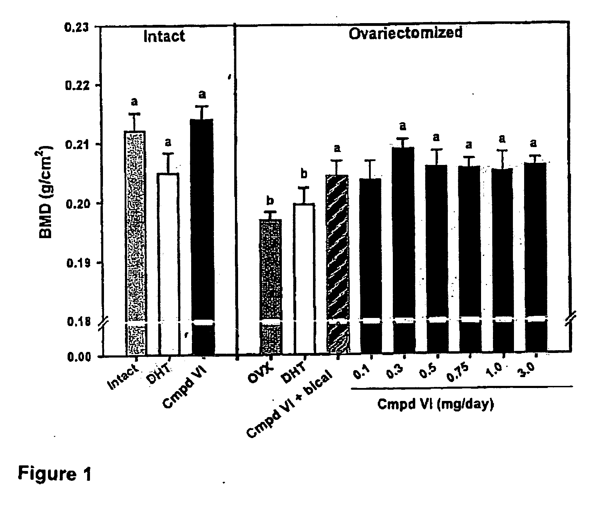

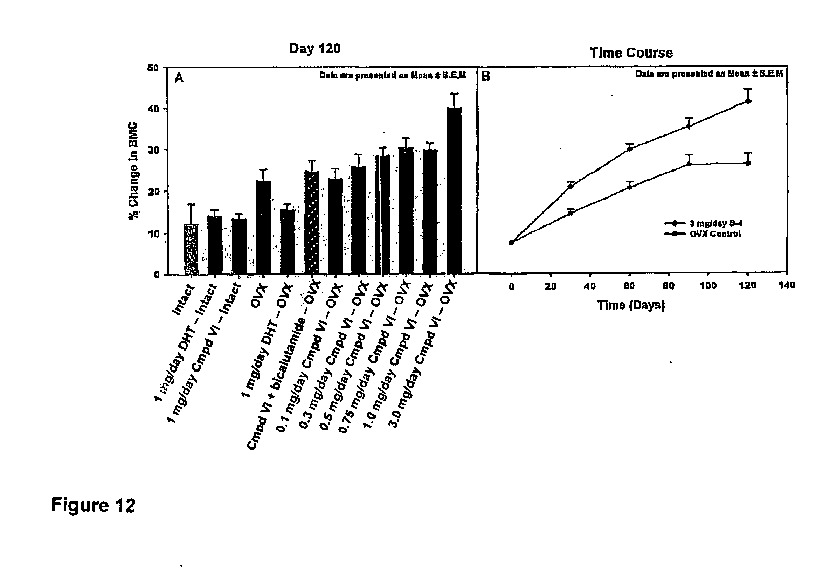

For example, the findings depicted in Figures 1-5 demonstrate that SARMS prevent loss of BMD, both overall in the body, and in a number of specific locations. These studies utilized the ovariectomized (OVX) rat model of osteoporosis, which has been shown to be highly predictive of success of osteoporosis therapy in humans (Kalu DN, Bone Miner 15: 175-91, 1991). Loss of BMD is a key indicator of osteoporosis, and is associated with decreased bone strength and increased fracture rate. By preventing loss in BMD, these and other symptoms of osteoporosis will be prevented as well. The findings depicted in Figures 12-13 show that SARMS increase BMC, another indicator of bone strength, in osteoporotic mice, verifying the findings of Figures 1-5.

In another embodiment, the present invention provides a method of increasing bone strength of a subject, comprising administering to the subject a SARM compound. In another embodiment, the method comprises administering an analogue, derivative, isomer, metabolite, pharmaceutically acceptable salt, pharmaceutical product, hydrate or N-oxide of the SARM compound, or any combination thereof.

In another embodiment, the present invention provides a method of increasing bone quality of a subject, comprising administering to the subject a SARM compound. In another embodiment, the method comprises administering an analogue, derivative, isomer, metabolite, pharmaceutically acceptable salt, pharmaceutical product, hydrate or N-oxide of the SARM compound, or any combination thereof.

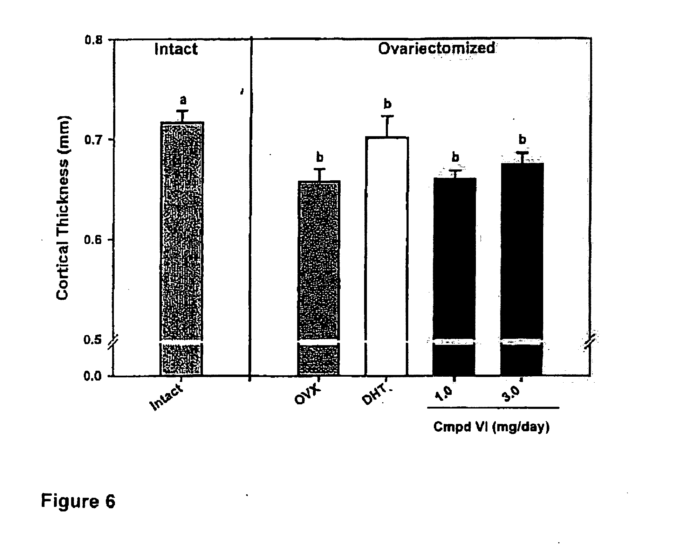

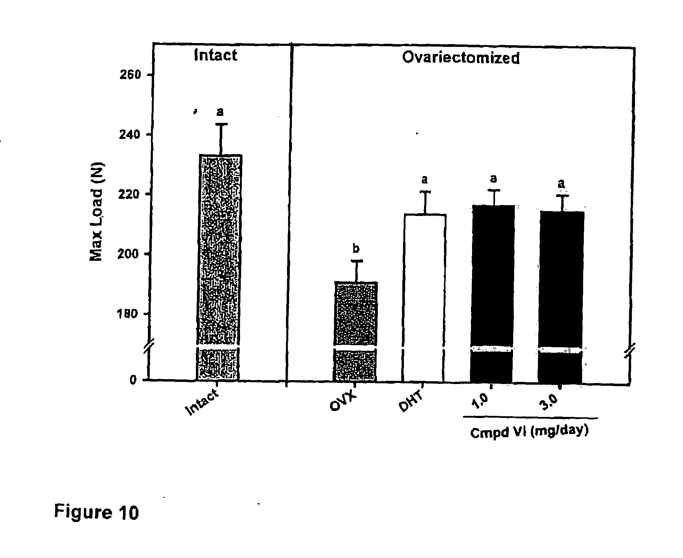

Methods for assessing bone mass, bone strength, and bone quality are well known in the art. For example, bone strength can be assessed, in one embodiment, using biomechanical testing (Figures 10, 11, and 24). Bone mass can be assessed, in one embodiment, using DEXA (Figures 1, 2, 4, 14, 15, 17-19, 25, and 26); or pQCT (Figures 6-9 and 20-23). Bone quality can be assessed by measuring BMC (Figures 12-13). Other methods for assessing bone mass and bone strength are described, for example in Faulkner KG et al (Am J Roentgenology 157: 1229-1237, 1991). Each method represents a separate embodiment of the present invention.

Similar results were obtained with the multiple means used in the present invention to measure bone mass, strength, and quality. The consistency of results between the different methods further validates the experimental results of the present invention.

In another embodiment, the present invention provides a method of increasing bone mass of a subject, comprising administering to the subject a SARM compound. In another embodiment, the method comprises administering an analogue, derivative, isomer, metabolite, pharmaceutically acceptable salt, pharmaceutical product, hydrate or N-oxide of the SARM compound, or any combination thereof.

In another embodiment, the present invention provides method of reducing the incidence of a bone resorption in a subject, comprising administering to the subject a SARM compound. In another embodiment, the method comprises administering an analogue, derivative, isomer, metabolite, pharmaceutically acceptable salt, pharmaceutical product, hydrate or N-oxide of the SARM compound, or any combination thereof.

In another embodiment, the present invention provides a method of preventing bone resorption in a subject, comprising administering one of the above compounds. In another embodiment, the present invention provides a method of suppressing bone resorption in a subject, comprising administering same. In another embodiment, the present invention provides a method of inhibiting bone resorption in a subject, comprising administering same.

Bone resorption is, in one embodiment, a major mechanism by which bone mass and/or bone strength is decreased as a result of disorders such as osteoporosis, menopause, and androgen deprivation. Methods of measuring bone resorption are well known in the art. For example, bone resorption can, in one embodiment, be measured by assessing serum osteocalcin levels (Example 8), which correlate with the level of bone resorption. In another embodiment, bone resorption can be assessed by measuring BMD (Figures 12-13). In another embodiment, bone resorption can be measured by assessing deoxypyridonoline levels in the urine. In another embodiment, bone resorption can be measured by assessing insulin-like growth factor (IGF-1) levels in the blood. Each method of assessing bone resorption represents a separate embodiment of the present invention.

In another embodiment, the term "bone resorption" refers to bone loss due to osteoclastic activity. Human bones are subject to a constant dynamic renovation process comprising bone resorption and bone formation. Bone resorption is based, in this embodiment, on the destruction of bone matrix by osteoclasts. The majority of bone disorders are based on a disturbed equilibrium between bone formation and bone resorption. Osteoporosis results from a deficit in new bone formation versus bone resorption during the ongoing remodeling process.

In one embodiment, the subject treated in the present invention has osteoporosis. In another embodiment, the subject has osteopenia. In another embodiment, the subject has increased bone resorption. In another embodiment, the subject has bone fracture. In another embodiment, the subject has bone frailty. In another embodiment, the subject has a loss of BMD. In another embodiment, the subject has any combination of osteoporosis, osteopenia, increased bone resorption, bone fracture, bone frailty and loss ofBMD. Each disorder represents a separate embodiment of the present invention.

For example, the findings presented in Figures 1-13 show that bone resorption, decreased BMD, and decreased bone strength as a result of ovariectomy was either partially or completely prevented by SARM treatment, depending on the area and type of bone assessed. Thus, SARMS are useful in reducing the incidence of bone resorption, decreased BMD, and decreased bone strength in a subject, as a result of, for example, osteoporosis, menopause, or any of the diseases or disorders described in the present invention.

In one embodiment, the subject treated in the present invention is a male subject. In another embodiment, the subject is an aging male subject. In another embodiment, the subject is a castrated male subject. In another embodiment, the subject is a man undergoing androgen-deprivation treatment. In another embodiment, the subject has prostate cancer. In another embodiment, the subject (male or female) has another type of cancer. In another embodiment, the subject is undergoing chemotherapy. In another embodiment, the subject has recently undergone chemotherapy.

In another embodiment, the subject is a female subject. In another embodiment, the subject is an aging female subject. In another embodiment, the subject is an HIV-positive premenopausal women. In another embodiment, the subject is a female having Addison's disease. In another embodiment, the subject is a female having a hypopituitary state. In another embodiment, the subject is an OVX female subject.

In another embodiment, the subject to whom the SARM compounds of the present invention are administered is an aging subject. The term "aging" means, in one embodiment, a process of becoming older. In another embodiment, the aging subject is a subject over 40 years old. In another embodiment, the aging subject is a subject over 45 years old. In another embodiment, the aging subject is a subject over 45 years old. In another embodiment, the aging the aging subject is a subject over 50 years old. In another embodiment, the aging subject is a subject over 55 years old. In another embodiment, the aging subject is a subject over 60 years old. In another embodiment, the aging subject is a subject over 65 years old. In another embodiment, the aging subject is a subject over 70 years old. Each type of subject represents a separate embodiment of the present invention.

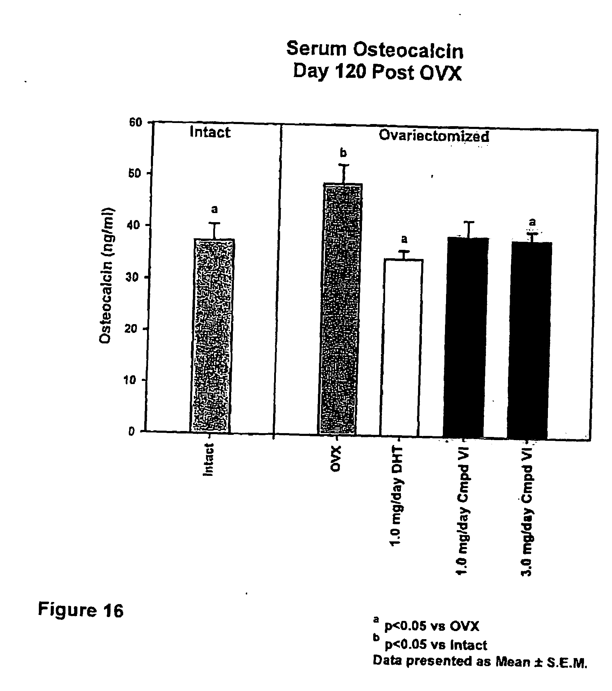

In another embodiment, the subject treated in the present invention does not have osteoporosis, osteopenia, increased bone resorption, bone fracture, bone frailty or loss of BMD. The findings presented in Figures 16-24 show that SARMS can reverse pre-existing loss of BMD and loss of bone strength resulting from osteoporosis. Thus, SARMS have anabolic activity independent of their ability to prevent bone resorption. Accordingly, the positive affects of SARMS on BMD, bone strength, and bone quality are by no means restricted to subjects that have experienced or are experiencing bone-related disorders; rather, the benefits of SARMS are applicable to any situation in which an increase in BMD, bone strength, or bone quality is desirable.

Accordingly, in another embodiment, the present invention provides a method of reversing loss of BMD in a subject, comprising administering a SARM or a metabolite or derivative thereof. In another embodiment, the present invention provides a method of reversing osteoporosis in a subject, comprising administering a SARM or a metabolite or derivative thereof. In another embodiment, the present invention provides a method of reversing osteopenia in a subject, comprising administering a SARM or a metabolite or derivative thereof. In another embodiment, the present invention provides a method of reversing bone frailty in a subject, comprising administering a SARM or a metabolite or derivative thereof. In one embodiment, the loss of BMD, osteoporosis, osteopenia, or bone frailty may be due to menopause or another hormonal disorder or imbalance. Each method represents a separate embodiment of the present invention.

There are several different types of bone in the skeleton, e.g, cortical bone and trabecular bone. Cortical bone serves as a protective covering and surrounds trabecular bone. Cortical bone has three layers, namely: the periosteal envelope (the outer surface of the bone); the intracortical envelope (the intermediate layer); and the endosteal envelope (the layer adjacent to the bone marrow cavity). Cortical bone is predominant in the limbs and is, in one embodiment, responsible for the skeleton's strength. Cortical bone can also be called, in one embodiment, Haversian or compact bone. Trabecular bone, which plays a role in bone metabolism, is also, in one embodiment, known as spongy or cancellous bone. The ratio of cortical and trabecular bone combination varies throughout the bones of the body.

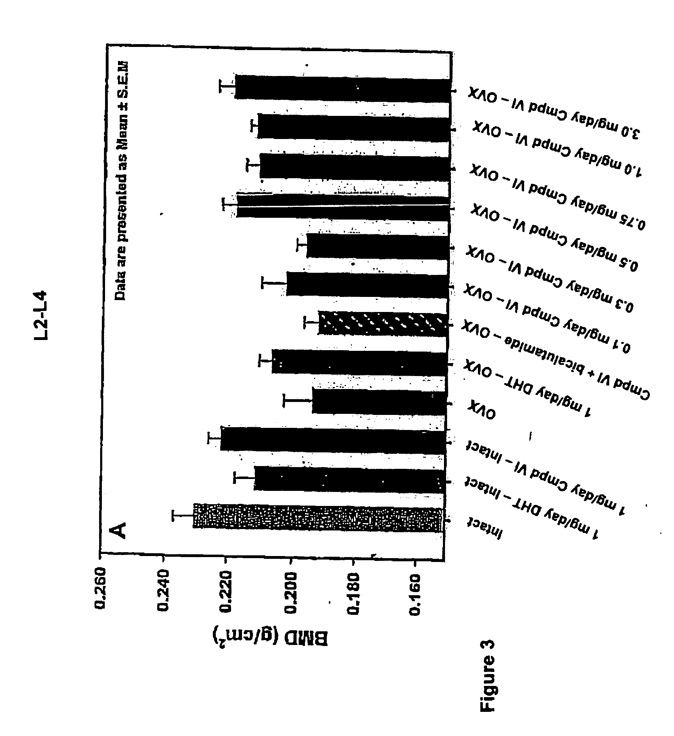

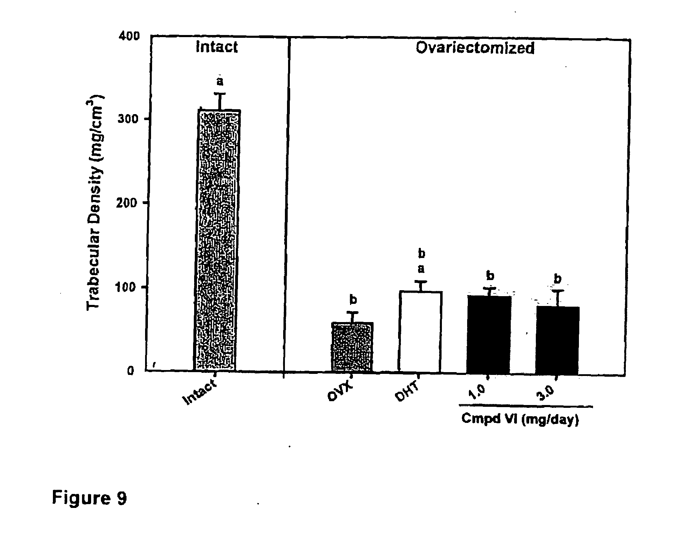

Thus, in one embodiment, the bone whose strength or mass is increased is cortical bone. The beneficial effects of SARMS on cortical bone are demonstrated in Figures 6-8 and 20-22. In another embodiment, the bone is trabecular bone. The beneficial effects of SARMS on trabecular bone are demonstrated in Figures 9 and 3. In another embodiment, the bone is cancellous bone. In another embodiment, the bone is Haversian bone. In another embodiment, the bone is intact bone comprising multiple types of bone tissue. In another embodiment, a particular layer of cortical bone may be affected by the methods of the present invention. In one embodiment, the layer is the periosteal envelope. In another embodiment, the layer is the intracortical envelope. In another embodiment, the layer is the endosteal envelope. Each type of bone represents a separate embodiment of the present invention.

In another embodiment, the present invention provides method of reducing an FM of a subject, comprising administering to the subject a SARM compound. In another embodiment, the method comprises administering an analogue, derivative, isomer, metabolite, pharmaceutically acceptable salt, pharmaceutical product, hydrate or N-oxide of the SARM compound, or any combination thereof.

In another embodiment, the present invention provides method of reducing an incidence of an increase in an FM of a subject, comprising administering to the subject a SARM compound. In another embodiment, the method comprises administering an analogue, derivative, isomer, metabolite, pharmaceutically acceptable salt, pharmaceutical product, hydrate or N-oxide of the SARM compound, or any combination thereof.

In another embodiment, the present invention provides method of increasing a muscle mass in a subject, comprising administering to the subject a SARM compound. In another embodiment, the method comprises administering an analogue, derivative, isomer, metabolite, pharmaceutically acceptable salt, pharmaceutical product, hydrate or N-oxide of the SARM compound, or any combination thereof.

In another embodiment, the present invention provides method of reducing an incidence of a decrease in a muscle mass in a subject, comprising administering to the subject a SARM compound. In another embodiment, the method comprises administering an analogue, derivative, isomer, metabolite, pharmaceutically acceptable salt, pharmaceutical product, hydrate or N-oxide of the SARM compound, or any combination thereof.

In another embodiment, the present invention provides method of increasing a lean mass in a subject, comprising administering to the subject a SARM compound. In another embodiment, the method comprises administering an analogue, derivative, isomer, metabolite, pharmaceutically acceptable salt, pharmaceutical product, hydrate or N-oxide of the SARM compound, or any combination thereof.

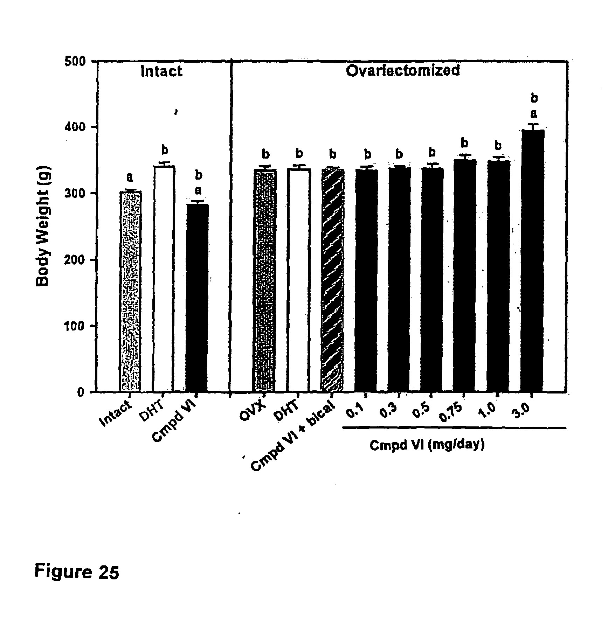

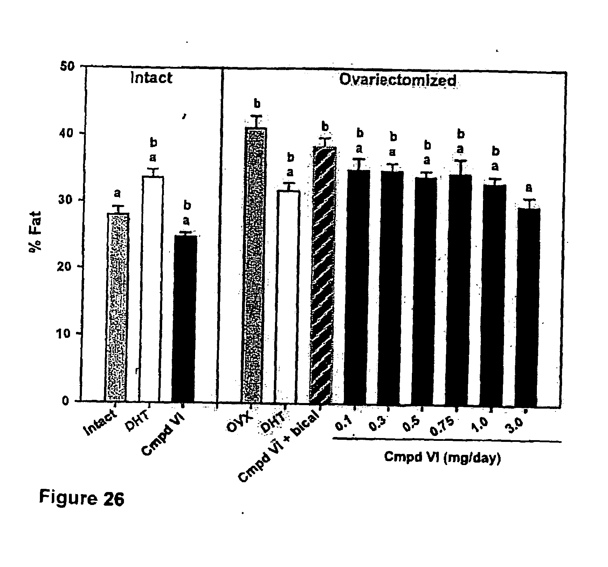

For example, the findings of Example 7 show that SARMS decrease the percentage of FM and increase the percentage of lean mass in OVX animals. Lean mass affects fracture risk for several reasons. First, increases in muscle mass are indirectly responsible for increases in BMD. Secondly, increasing muscle mass may improve balance and muscle strength, thereby reducing the risk of falling, which is a primary cause of fracture in the elderly. Thus, in another embodiment, the present invention provides a method of decreasing fracture risk, via increasing muscle mass. In another embodiment, the present invention provides a method of decreasing fracture risk, via decreasing FM. Moreover, the findings depicted in Figure 26 show that SARMS are able to reverse an existing increase in FM. Combined with the body weight studies depicted in Figure 25, these findings show a reversal of an existing decrease in lean mass. Accordingly, the positive affects of SARMS on FM, muscle mass, and lean mass are by no means restricted to subjects experiencing bone-related disorders, but rather are applicable to any situation in which a subject wishes to increase FM, muscle mass, or lean mass.

"FM" refers, in one embodiment, to the amount of total fat in the subject's body. In another embodiment, "FM" refers to the percentage body fat of the subject. In another embodiment, FM refers to the amount of total fat or percentage body fat in a particular area of the body. In another embodiment, FM refers to the amount or percentage of a particular type of fat. Each type of FM represents a separate embodiment of the present invention.

In one embodiment, the fat affected by the present invention is subcutaneous fat. In another embodiment, the fat is trunk fat. In another embodiment, the fat is intra-abdominal fat. In another embodiment, the fat is any other type of fat known in the art. Each type of fat represents a separate embodiment of the present invention.

Decreasing FM and increasing lean mass and/or muscle mass has, in one embodiment, a positive effect on impaired glucose metabolism. In another embodiment, decreasing FM and increasing lean mass and/or muscle mass has a positive effect on diabetes. In another embodiment, decreasing FM and increasing lean mass and/or muscle mass has a positive effect on hypertension. In another embodiment, decreasing FM and increasing lean mass and/or muscle mass has a positive effect on coronary disease. In another embodiment, decreasing FM and increasing lean mass and/or muscle mass has a positive effect on obesity. In another embodiment, decreasing FM and increasing lean mass and/or muscle mass has a positive effect on a disease or disorder associated with impaired glucose metabolism, diabetes, hypertension, coronary disease, or obesity. Thus, in another embodiment, the present invention provides a means of treating or ameliorating a impaired glucose metabolism, diabetes, hypertension, coronary disease, obesity, or an associated disease or disorder, comprising administration of a SARM or a derivative or metabolite thereof.

### Selective Androgen Receptor Modulators:

The SARM compounds of the present invention are, in one embodiment, a novel class of AR targeting agents that demonstrate androgenic or anti-androgenic and anabolic activity. In another embodiment, the SARM compounds of the present invention are a novel class of non-steroidal ligands for the AR.

In another embodiment, the SARM compounds of the present invention may be categorized into subgroups depending on their biological activity. For example, several SARM compounds have an agonistic effect on muscle or bone, whereas others have an antagonistic effect.

The AR is a ligand-activated transcriptional regulatory protein that mediates induction of male sexual development and function through its activity with endogenous androgens (male sex hormones). The androgens (e.g. DHT and testosterone) are steroids that are produced in the body by the testis and the cortex of the adrenal gland. Thus, in one embodiment, SARMS are AR ligands that differ from previously known AR ligands in that SARMS are non-steroidal.

A receptor agonist is, in one embodiment, a substance that binds a receptor and activates it. A receptor partial agonist is, in one embodiment, a substance that binds a receptor and partially activates it. A receptor antagonist is, in one embodiment, a substance that binds a receptor and inactivates it. In one embodiment, the SARM compounds of the present invention have a tissue-selective effect, wherein one agent may be an agonist, partial agonist and/or antagonist, depending on the tissue. For example, the SARM compound may stimulate muscle tissue and at the same time inhibit prostate tissue. In one embodiment, the SARMs of the present invention are AR agonists. In another embodiment, the SARMs are AR antagonists. Assays to determine whether the compounds of the present invention are AR agonists or antagonists are well known to a person skilled in the art. For example, AR agonistic activity can be determined by monitoring the ability of the SARM compounds to maintain and/or stimulate the growth of AR containing tissue such as prostate and seminal vesicles, as measured by weight. AR antagonistic activity can be determined by monitoring the ability of the SARM compounds inhibit the growth of AR containing tissue.

In another embodiment, the SARM compounds of the present invention can be classified as partial AR agonist/antagonists. The SARMs are AR agonists in some tissues, causing increased transcription of AR-responsive genes (e.g. muscle anabolic effect). In other tissues, these compounds serve as competitive inhibitors of testosterone and/or dihydrotestosterone (DHT) on the AR to prevent agonistic effects of the native androgens. Each type of SARM represents a separate embodiment of the present invention.

In one embodiment, the SARM compounds of the present invention bind reversibly to the AR. In another embodiment, the SARM compounds bind irreversibly to the AR. The compounds of the present invention may, in one embodiment, contain a functional group (affinity label) that allows alkylation of the AR (i.e. covalent bond formation). Thus, in this case, the compounds bind irreversibly to the receptor and, accordingly, cannot be displaced by a steroid, such as the endogenous ligands DHT and testosterone.

In one embodiment of the present invention, the SARM compound is administered to the subject. In another embodiment, an analogue of the SARM is administered. In another embodiment, a derivative of the SARM is administered. In another embodiment, an isomer of the SARM is administered. In another embodiment, a metabolite of the SARM is administered. In another embodiment, a pharmaceutically acceptable salt of the SARM is administered. In another embodiment, a pharmaceutical product of the SARM is administered. In another embodiment, a hydrate of the SARM is administered. In another embodiment, an N-oxide of the SARM is administered. In another embodiment, the methods of the present invention comprise administering any of a combination of an analogue, derivative, isomer, metabolite, pharmaceutically acceptable salt, pharmaceutical product, hydrate or N-oxide of the SARM. Each possibility represents a separate embodiment of the present invention.

The term "isomer" refers, in one embodiment, an optical isomer. In another embodiment, "isomer" refers to an analog. In another embodiment, "isomer" refers to a structural isomer. In another embodiment, "isomer" refers to a structural analog. In another embodiment, "isomer" refers to a conformational isomer. In another embodiment, "isomer" refers to a conformational analog. In another embodiment, "isomer" refers to any other type of isomer known in the art. Each type of isomer represents a separate embodiment of the present invention.

In another embodiment, this invention encompasses the use of various optical isomers of the SARM compound. It will be appreciated by those skilled in the art that the SARMs of the present invention contain at least one chiral center. Accordingly, the SARMs used in the methods of the present invention may exist in, and be isolated in, optically active or racemic forms. Some compounds may also exhibit polymorphism. It is to be understood that the present invention encompasses any racemic, optically active, polymorphic, or stereroisomeric form, or mixtures thereof, which form possesses properties useful in the treatment of androgen-related conditions described herein. In one embodiment, the SARMs are the pure (R)-isomers. In another embodiment, the SARMs are the pure (S)-isomers. In another embodiment, the SARMs are a mixture of the (R) and (S) isomers. In another embodiment, the SARMs are a racemic mixture comprising an equal amount of the (R) and (S) isomers. It is well known in the art how to prepare optically-active forms (for example, by resolution of the racemic form by recrystallization techniques, by synthesis from optically-active starting materials, by chiral synthesis, or by chromatographic separation using a chiral stationary phase).

The invention includes, in another embodiment, pharmaceutically acceptable salts of amino-substituted compounds with organic and inorganic acids, for example, citric acid and hydrochloric acid. The invention also includes N-oxides of the amino substituents of the compounds described herein. Pharmaceutically acceptable salts can also be prepared from the phenolic compounds by treatment with inorganic bases, for example, sodium hydroxide. Also, esters of the phenolic compounds can be made with aliphatic and aromatic carboxylic acids, for example, acetic acid and benzoic acid esters.

This invention further includes, in another embodiment, derivatives of the SARM compounds. The term "derivatives" includes but is not limited to ether derivatives, acid derivatives, amide derivatives, ester derivatives and the like. In addition, this invention further includes hydrates of the SARM compounds. The term "hydrate" includes but is not limited to hemi-hydrate, monohydrate, dihydrate, trihydrate and the like.

This invention further includes, in another embodiment, metabolites of the SARM compounds. The term "metabolite" refers, in one embodiment, to any substance produced from another substance by metabolism or a metabolic process.

This invention further includes, in one embodiment, pharmaceutical products of the SARM compounds. The term "pharmaceutical product" refers, in one embodiment, to a composition suitable for pharmaceutical use (pharmaceutical composition), as defined herein.

In one embodiment, the SARM compound of the present invention is a compound represented by the structure of formula I:

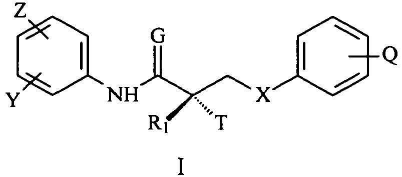

In another embodiment, the SARM compound of the present invention is a compound represented by the structure of formula II: 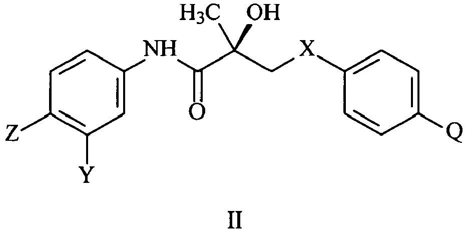 wherein X is a bond, O, CH₂, NH, Se, PR, NO or NR;
Z is NO₂, CN, COOH, COR, NHCOR or CONHR;
Y is CF₃, F, I, Br, Cl, CN, CR₃ or SnR₃;
Q is alkyl, F, I, Br, Cl, CF₃, CN CR₃, SnR₃, NR₂, NHCOCH₃, NHCOCF₃, NHCOR, NHCONHR, NHCOOR, OCONHR, CONHR, NHCSCH₃, NHCSCF₃, NHCSR NHSO₂CH₃, NHSO₂R, OR, COR, OCOR, OSO₂R, SO₂R, SR, SCN, NCS, OCN, NCO; or Q together with the benzene ring to which it is attached is a fused ring system represented by structure A, B or C: 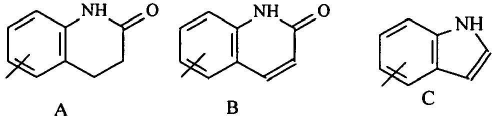 R is alkyl, haloalkyl, dihaloalkyl, trihaloalkyl, CH₂F, CHF₂, CF₃, CF₂CF₃, aryl, phenyl, F, I, Br, Cl, alkenyl or OH.

In one embodiment, the SARM compound is a compound of formula II wherein X is O. In another embodiment, the SARM compound is a compound of formula II wherein Z is NO₂. In another embodiment, the SARM compound is a compound of formula II wherein Z is CN. In another embodiment, the SARM compound is a compound of formula II wherein Y is CF₃. In another embodiment, the SARM compound is a compound of formula II wherein Q is NHCOCH₃. In another embodiment, the SARM compound is a compound of formula II wherein Q is F.

In one embodiment, the substituent R in compound (I) or (II) is an alkyl group. In another embodiment, the substituent R is a haloalkyl group. In another embodiment, the substituent R is a dihaloalkyl group. In another embodiment, the substituent R is a trihaloalkyl group. In another embodiment, the substituent R is a CH₂F moiety. In another embodiment, the substituent R is a CHF₂ moiety. In another embodiment, the substituent R is a CF₃ moiety. In another embodiment, the substituent R is a CF₂CF₃ moiety. In another embodiment, the substituent R is an aryl group. In another embodiment, the substituent R is a phenyl group. In another embodiment, the substituent R is F. In another embodiment, the substituent R is I. In another embodiment, the substituent R is a Br. In another embodiment, the substituent R is Cl. In another embodiment, the substituent R is an alkenyl group. In another embodiment, the substituent R is an OH moiety. Each substituent represents a separate embodiment of the present invention.

In another embodiment, the SARM compound of the present invention is a compound represented by the structure of formula III: 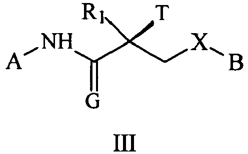 wherein X is a bond, O, CH₂, NH, Se, PR, NO or NR;
G is O or S;
R₁ is CH₃, CH₂F, CHF₂, CF₃, CH₂CH₃, or CF₂CF₃;
T is OH, OR, -NHCOCH₃, or NHCOR;
R is alkyl, haloalkyl, dihaloalkyl, trihaloalkyl, CH₂F, CHF₂, CF₃, CF₂CF₃, aryl, phenyl, F, I, Br, Cl, alkenyl or OH;
A is a ring selected from: 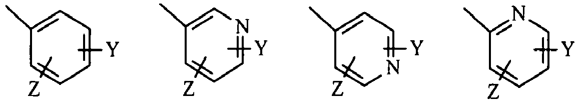 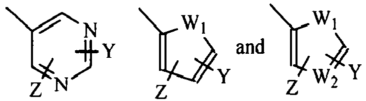 B is a ring selected from: 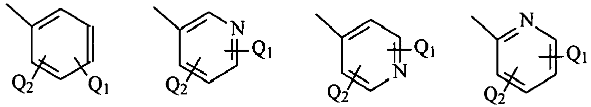 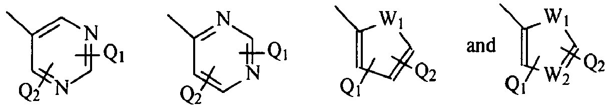 wherein A and B cannot simultaneously be a benzene ring;
Z is NO₂, CN, COOH, COR, NHCOR or CONHR;
Y is CF₃, F, I, Br, Cl, CN CR₃ or SnR₃;
Q₁ and Q₂ are independently of each other a hydrogen, alkyl, F, I, Br, Cl, CF₃, CN CR₃, SnR₃, NR₂, NHCOCH₃, NHCOCF₃, NHCOR, NHCONHR, NHCOOR, OCONHR, CONHR, NHCSCH₃, NHCSCF₃, NHCSR NHSO₂CH₃, NHSO₂R, OR, COR, OCOR, OSO₂R SO₂R, SR, SCN, NCS, OCN, NCO, 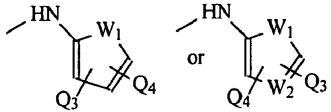 Q₃ and Q₄ are independently of each other a hydrogen, alkyl, F, I, Br, Cl, CF₃, CN CR₃, SnR₃, NR₂, NHCOCH₃, NHCOCF₃, NHCOR, NHCONHR, NHCOOR, OCONHR, CONHR, NHCSCH₃, NHCSCF₃, NHCSR NHSO₂CH₃, NHSO₂R, OR, COR, OCOR, OSO₂R, SO₂R, SR, SCN, NCS, OCN, or NCO;
W₁ is O, NH, NR, NO or S; and
W₂ is N or NO.

In one embodiment, the SARM compound is a compound of formula III wherein X is O. In another embodiment, the SARM compound is a compound of formula III wherein G is O. In another embodiment, the SARM compound is a compound of formula I wherein T is OH. In another embodiment, the SARM compound is a compound of formula III wherein R₁ is CH₃. In another embodiment, the SARM compound is a compound of formula III wherein Z is NO₂. In another embodiment, the SARM compound is a compound of formula III wherein Z is CN. In another embodiment, the SARM compound is a compound of formula III wherein Y is CF₃. In another embodiment, the SARM compound is a compound of formula III wherein Q₁ is NHCOCH₃. In another embodiment, the SARM compound is a compound of formula III wherein Q₁ is F.

The substituents Z and Y can be, in one embodiment, in any position of the ring carrying these substituents (hereinafter "A ring"). In one embodiment, the substituent Z is in the para position of the A ring. In another embodiment, the substituent Y is in the meta position of the A ring. In another embodiment, the substituent Z is in the para position of the A ring and substituent Y is in the meta position of the A ring.

The substituents Q₁ and Q₂ can be, in one embodiment, in any position of the ring carrying these substituents (hereinafter "B ring"). In one embodiment, the substitutent Q₁ is in the para position of the B ring. In another embodiment, the subsituent is Q₂ is H. In another embodiment, the substitutent Q₁ is in the para position of the B ring and the subsituent is Q₂ is H. In another embodiment, the substitutent Q₁ is NHCOCH₃ and is in the para position of the B ring, and the substituent is Q₂ is H.

Each substituent of each of the above variables represents a separate embodiment of the present invention. Further, each position enumerated above of each of the above substituents represents a separate embodiment of the present invention.

In another embodiment, the SARM compound of the present invention is a compound represented by the structure of formula IV: 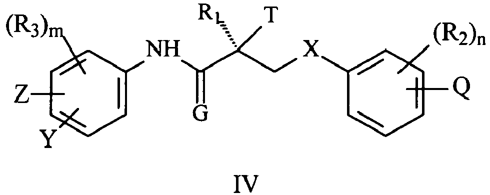 wherein X is a bond, O, CH₂, NH, Se, PR, NO or NR;
G is O or S;
T is OH, OR, -NHCOCH₃, or NHCOR;
R is alkyl, haloalkyl, dihaloalkyl, trihaloalkyl, CH₂F, CHF₂, CF₃,
CF₂CF₃, aryl, phenyl, F, I, Br, Cl, alkenyl or OH;
R₁ is CH₃, CH₂F, CHF₂, CF₃, CH₂CH₃, or CF₂CF₃;
R₂ is F, Cl, Br, I, CH₃, CF₃, OH, CN, NO₂, NHCOCH₃, NHCOCF₃, NHCOR, alkyl, arylalkyl, OR, NH₂, NHR, NR₂, SR, SCN, NCS, OCN, NCO;
R₃ is F, Cl, Br, I, CN, NO₂, COR, COOH, CONHR, CF₃, SnR₃, or R₃ together with the benzene ring to which it is attached forms a fused ring system represented by the structure: 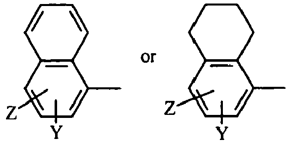 Z is NO₂, CN, COR, COOH, or CONHR;
Y is CF₃, F, Br, Cl, I, CN, or SnR₃;
Q is H, alkyl, F, I, Br, Cl, CF₃, CN CR₃, SnR₃, NR₂, NHCOCH₃, NHCOCF₃, NHCOR, NHCONHR, NHCOOR, OCONHR, CONHR, NHCSCH₃, NHCSCF₃, NHCSR NHSO₂CH₃, NHSO₂R, OH, OR, COR, OCOR, OSO₂R, SO₂R, SR; or Q together with the benzene ring to which it is attached is a fused ring system represented by structure A, B or C: 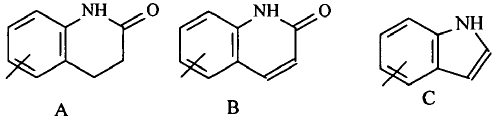 n is an integer of 1-4; and
m is an integer of 1-3.

In one embodiment, the SARM compound is a compound of formula IV wherein X is O. In another embodiment, the SARM compound is a compound of formula IV wherein G is O. In another embodiment, the SARM compound is a compound of formula IV wherein Z is NO₂. In another embodiment, the SARM compound is a compound of formula IV wherein Z is CN. In another embodiment, the SARM compound is a compound of formula IV wherein Y is CF₃. In another embodiment, the SARM compound is a compound of formula IV wherein Q is NHCOCH₃. In another embodiment, the SARM compound is a compound of formula IV wherein Q is F. In another embodiment, the SARM compound is a compound of formula IV wherein T is OH. In another embodiment, the SARM compound is a compound of formula IV wherein R₁ is CH₃. In another embodiment, the SARM compound is a compound of formula IV wherein Q is F and R₂ is CH₃. In another embodiment, the SARM compound is a compound of formula IV wherein Q is F and R₂ is Cl.

The substituents Z, Y, and R₃ can be, in one embodiment, in any position of the ring carrying these substituents (hereinafter "A ring"). In one embodiment, the substituent Z is in the para position of the A ring. In another embodiment, the substituent Y is in the meta position of the A ring. In another embodiment, the substituent Z is in the para position of the A ring and substituent Y is in the meta position of the A ring.

The substituents Q and R₂ can be, in one embodiment, in any position of the ring carrying these substituents (hereinafter "B ring"). In one embodiment, the substitutent Q is in the para position of the B ring. In another embodiment, the substitutent Q is in the para position of the B ring. In another embodiment, the substitutent Q is NHCOCH₃ and is in the para position of the B ring.

In one embodiment, when the integers m and n are greater than one, the substituents R₂ and R₃ are not limited to one particular substituent, and can be any combination of the substituents listed above.

Each substituent of each of the above variables represents a separate embodiment of the present invention. Further, each position enumerated above of each of the above substituents represents a separate embodiment of the present invention. Further, each number enumerated above of each of the above integers represents a separate embodiment of the present invention.

In another embodiment, the SARM compound of the present invention is a compound represented by the structure of formula V: 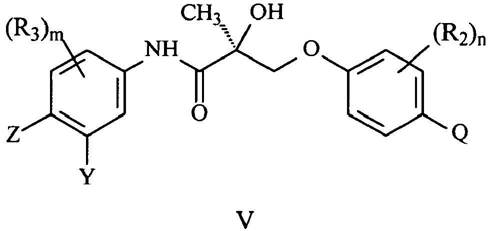 wherein
R₂ is F, Cl, Br, I, CH₃, CF₃, OH, CN, NO₂, NHCOCH₃, NHCOCF₃, NHCOR, alkyl, arylalkyl, OR, NH₂, NHR, NR₂, SR;
R₃ is F, Cl, Br, I, CN, NO₂, COR, COOH, CONHR, CF₃, SnR₃, or R₃ together with the benzene ring to which it is attached forms a fused ring system represented by the structure: 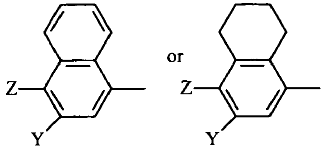 R is alkyl, haloalkyl, dihaloalkyl, trihaloalkyl, CH₂F, CHF₂, CF₃, CF₂CF₃, aryl, phenyl, F, I, Br, Cl, alkenyl or OH;
Z is NO₂, CN, COR, COOH, or CONHR;
Y is CF₃, F, Br, Cl, I, CN, or SnR₃;
Q is H, alkyl, F, I, Br, Cl, CF₃, CN CR₃, SnR₃, NR₂, NHCOCH₃, NHCOCF₃, NHCOR, NHCONHR, NHCOOR, OCONHR, CONHR, NHCSCH₃, NHCSCF₃, NHCSR NHSO₂CH₃, NHSO₂R, OH, OR, COR, OCOR, OSO₂R, SO₂R, SR; or Q together with the benzene ring to which it is attached is a fused ring system represented by structure A, B or C: 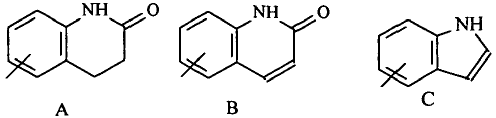 n is an integer of 1-4; and
m is an integer of 1-3.

In another embodiment, the SARM is a compound of formula V wherein Z is NO₂. In another embodiment, the SARM is a compound of formula V wherein Z is CN. In another embodiment, the SARM is a compound of formula V wherein Y is CF₃. In another embodiment, the SARM is a compound of formula V wherein Q is NHCOCH₃. In another embodiment, the SARM is a compound of formula V wherein Q is F. In another embodiment, the SARM is a compound of formula V wherein Q is F and R₂ is CH₃. In another embodiment, the SARM is a compound of formula V wherein Q is F and R₂ is Cl.

The substituents Z, Y and R₃ can be in, in one embodiment, any position of the A ring, and the substituents Q and R₂ can be, in one embodiment, in any position ofB ring, as discussed above for compound IV. Furthermore, as discussed above, when the integers m and n are greater than one, the substituents R₂ and R₃ are not limited to one particular substituent, and can be any combination of the substituents listed above.

Each substituent of each of the above variables represents a separate embodiment of the present invention. Further, each position enumerated above of each of the above substituents represents a separate embodiment of the present invention. Further, each number enumerated above of each of the above integers represents a separate embodiment of the present invention.

In another embodiment, the SARM compound of the present invention is a compound represented by the structure of formula VI.

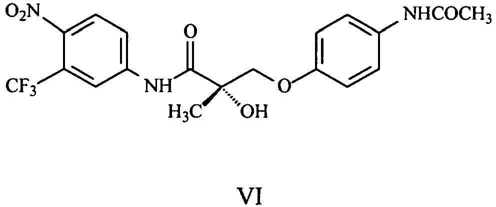

The findings of the present invention show that Compound VI has beneficial effects on bone, fat, and muscle tissue, as delineated above. In addition, the pharmaco-kinetic properties of Compound VI favor its use as a pharmaceutical treatment of conditions disclosed in the present invention. The present invention has shown that Compound VI exhibits decreased metabolic clearance and enhanced oral bioavailability compared to testosterone. These properties, as well as rapidly reaching peak plasma concentration, were observed with Compound VI at doses capable of eliciting maximal pharmacologic effect (Example 15).

In another embodiment, the SARM compound of the present invention is a compound represented by the structure of formula VII.

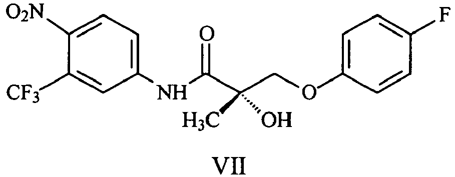

In another embodiment, the SARM compound of the present invention is a compound represented by the structure of formula VIII.

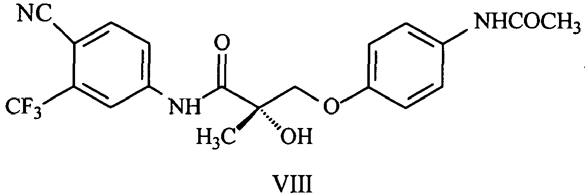

In another embodiment, the SARM compound of the present invention is a compound represented by the structure of formula IX.

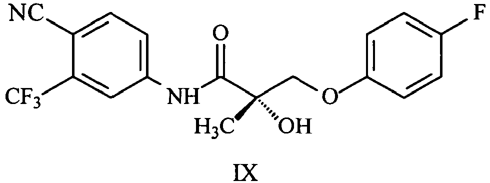

In another embodiment, the SARM compound of the present invention is a compound represented by the structure of formula X.

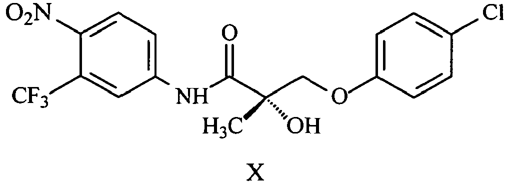

In another embodiment, the SARM compound of the present invention is a compound represented by the structure of formula XI.

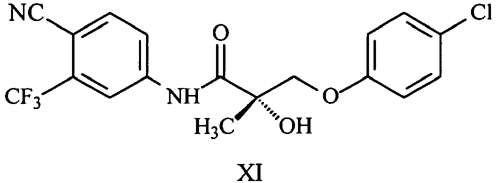

In another embodiment, the SARM compound is a compound represented by a structure of formula XII:

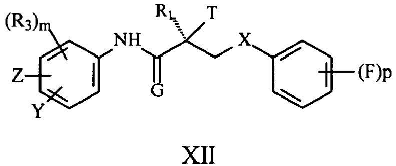

In one embodiment, p is 2. In another embodiment, p is 3. In another embodiment, p is 4. In another embodiment, p is 5. The rest of the substituents are as defined above for formula IV.

In another embodiment, the SARM compound is a compound represented by a structure of formula XIII:

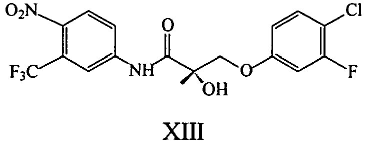

In another embodiment, the SARM compound is a compound represented by a structure of formula XIV:

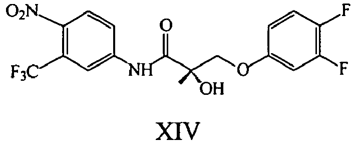

In another embodiment, the SARM compound is a compound represented by a structure of formula XV:

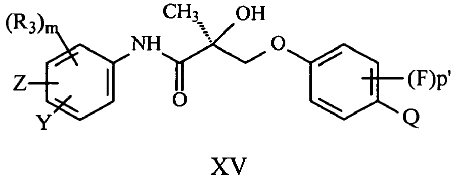

In another embodiment, p is 1. In one embodiment, p is 2. In another embodiment, p is 3. In another embodiment, p is 4. The rest of the substituents are as defined above for formula V.

In another embodiment, the SARM compound is a compound represented by a structure of formula XVI:

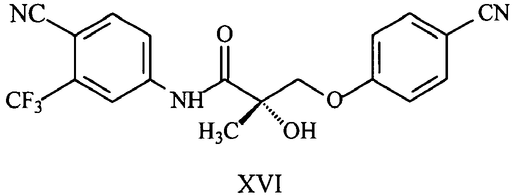

In another embodiment, the SARM compound is a compound represented by a structure of formula XVII:

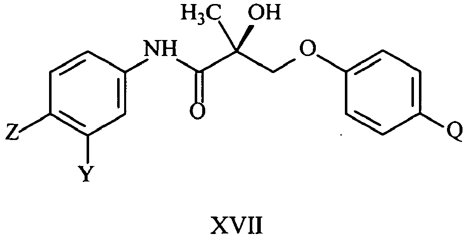

In one embodiment, the SARM is a compound of formula XVII wherein Q is acetamido (NHCOCH₃). In another embodiment, the SARM is a compound of formula XVII wherein Q is trifluoroacetamido (NHCOCF₃).

In another embodiment, the SARM is a compound of formula XVII wherein Z is NO₂. In another embodiment, the SARM is a compound of formula XVII wherein Z is CN. In another embodiment, the SARM is a compound of formula XVII wherein Z is COR. In another embodiment, the SARM is a compound of formula XVII wherein Z is CONHR.

In another embodiment, the SARM is a compound of formula XVII wherein Y is CF₃. In another embodiment, the SARM is a compound of formula XVII wherein Y is I. In another embodiment, the SARM is a compound of formula XVII wherein Y is Br. In another embodiment, the SARM is a compound of formula XVII wherein Y is Cl. In another embodiment, the SARM is a compound of formula XVII wherein Y is SnR₃.

In another embodiment, the SARM is a compound of formula XVII wherein R is an alkyl group. In another embodiment, the SARM is a compound of formula XVII wherein R is OH.

Each substituent of each of the above variables represents a separate embodiment of the present invention. Further, each position enumerated above of each of the above substituents represents a separate embodiment of the present invention.

In another embodiment, the SARM compound is a compound represented by a structure of formula XVIII: 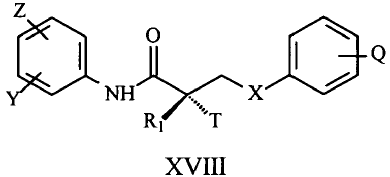 wherein
X is O, CH₂, NH, Se, PR, NO or NR;
T is OH, OR, -NHCOCH₃, or NHCOR;
Z is NO₂, CN, COOH, COR, NHCOR or CONHR;
Y is CF₃, F, I, Br, Cl, CN, CR₃ or SnR₃;
Q is alkyl, F, I, Br, Cl, CF₃, CN, CR₃, SnR₃, NR₂, NHCOCH₃, NHCOCF₃, NHCOR, NHCONHR, NHCOOR, OCONHR, CONHR, NHCSCH₃, NHCSCF₃, NHCSR, NHSO₂CH₃, NHSO₂R, OR, COR, OCOR, OSO₂R, SO₂R, SR; or Q together with the benzene ring to which it is attached is a fused ring system represented by structure A, B or C: 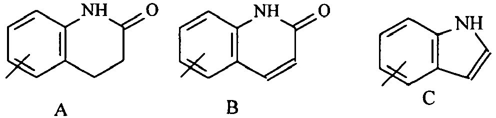 R is alkyl, haloalkyl, dihaloalkyl, trihaloalkyl, CH₂F, CHF₂, CF₃, CF₂CF₃, aryl, phenyl, F, I, Br, Cl, alkenyl or OH; and
R₁ is CH₃, CH₂F, CHF₂, CF₃, CH₂CH₃, or CF₂CF₃.

Each substituent of each of the above variables represents a separate embodiment of the present invention. Further, each position enumerated above of each of the above substituents represents a separate embodiment of the present invention.

In one embodiment, the SARM compound is a compound of one of the above formulas wherein X is O. In another embodiment, the SARM compound is a compound of one of the above formulas wherein X is a bond. In another embodiment, the SARM compound is a compound of one of the above formulas wherein X is CH₂. In another embodiment, the SARM compound is a compound of one of the above formulas wherein X is NH. In another embodiment, the SARM compound is a compound of one of the above formulas wherein X is Se. In another embodiment, the SARM compound is a compound of one of the above formulas wherein X is PR. In another embodiment, the SARM compound is a compound of one of the above formulas wherein X is NO. In another embodiment, the SARM compound is a compound of one of the above formulas wherein X is NR.

In one embodiment, the SARM compound is a compound of one of the above formulas wherein G is O. In another embodiment, the SARM compound is a compound of one of the above formulas wherein G is S.

In one embodiment, the SARM compound is a compound of one of the above formulas wherein T is OH. In another embodiment, the SARM compound is a compound of one of the above formulas wherein T is OR. In another embodiment, the SARM compound is a compound of one of the above formulas wherein -NHCOCH₃. In another embodiment, the SARM compound is a compound of one of the above formulas wherein T is NHCOR.

In another embodiment, the SARM compound is a compound of one of the above formulas wherein Z is NO₂. In another embodiment, the SARM compound is a compound of one of the above formulas wherein Z is CN. In another embodiment, the SARM compound is a compound of one of the above formulas wherein Z is COOH. In another embodiment, the SARM compound is a compound of one of the above formulas wherein Z is COR. In another embodiment, the SARM compound is a compound of one of the above formulas wherein Z is NHCOR. In another embodiment, the SARM compound is a compound of one of the above formulas wherein Z is CONHR.

In another embodiment, the SARM compound is a compound of one of the above formulas wherein Y is CF₃. In another embodiment, the SARM compound is a compound of one of the above formulas wherein Y is F. In another embodiment, the SARM compound is a compound of one of the above formulas wherein Y is I. In another embodiment, the SARM compound is a compound of one of the above formulas wherein Y is Br. In another embodiment, the SARM compound is a compound of one of the above formulas wherein Y is Cl. In another embodiment, the SARM compound is a compound of one of the above formulas wherein Y is CN. In another embodiment, the SARM compound is a compound of one of the above formulas wherein Y is CR₃. In another embodiment, the SARM compound is a compound of one of the above formulas wherein Y is SnR₃.

In another embodiment, the SARM compound is a compound of one of the above formulas wherein Q is NHCOCH₃. In another embodiment, the SARM compound is a compound of one of the above formulas wherein Q is F. In another embodiment, the SARM compound is a compound of one of the above formulas wherein Q is alkyl. In another embodiment, the SARM compound is a compound of one of the above formulas wherein Q is I. In another embodiment, the SARM compound is a compound of one of the above formulas wherein Q is Br. In another embodiment, the SARM compound is a compound of one of the above formulas wherein Q is Cl. In another embodiment, the SARM compound is a compound of one of the above formulas wherein Q is CF₃. In another embodiment, the SARM compound is a compound of one of the above formulas wherein Q is CN. In another embodiment, the SARM compound is a compound of one of the above formulas wherein Q is CR₃. In another embodiment, the SARM compound is a compound of one of the above formulas wherein Q is SnR₃. In another embodiment, the SARM compound is a compound of one of the above formulas wherein Q is NR₂. In another embodiment, the SARM compound is a compound of one of the above formulas wherein Q is NHCOCF₃. In another embodiment, the SARM compound is a compound of one of the above formulas wherein Q is NHCOR. In another embodiment, the SARM compound is a compound of one of the above formulas wherein Q is NHCONHR. In another embodiment, the SARM compound is a compound of one of the above formulas wherein Q is NHCOOR. In another embodiment, the SARM compound is a compound of one of the above formulas wherein Q is OCONHR. In another embodiment, the SARM compound is a compound of one of the above formulas wherein Q is CONHR. In another embodiment, the SARM compound is a compound of one of the above formulas wherein Q is NHCSCH₃. In another embodiment, the SARM compound is a compound of one of the above formulas wherein Q is NHCSCF₃. In another embodiment, the SARM compound is a compound of one of the above formulas wherein Q is NHCSR. In another embodiment, the SARM compound is a compound of one of the above formulas wherein Q is NHSO₂CH₃. In another embodiment, the SARM compound is a compound of one of the above formulas wherein Q is NHSO₂R. In another embodiment, the SARM compound is a compound of one of the above formulas wherein Q is OR. In another embodiment, the SARM compound is a compound of one of the above formulas wherein Q is COR. In another embodiment, the SARM compound is a compound of one of the above formulas wherein Q is OCOR. In another embodiment, the SARM compound is a compound of one of the above formulas wherein Q is OSO₂R. In another embodiment, the SARM compound is a compound of one of the above formulas wherein Q is SO₂R. In another embodiment, the SARM compound is a compound of one of the above formulas wherein Q is SR. In another embodiment, the SARM compound is a compound of one of the above formulas wherein Q is SCN. In another embodiment, the SARM compound is a compound of one of the above formulas wherein Q is NCS. In another embodiment, the SARM compound is a compound of one of the above formulas wherein Q is OCN. In another embodiment, the SARM compound is a compound of one of the above formulas wherein Q is NCO.

In another embodiment, the SARM compound is a compound of one of the above formulas wherein Q together with the benzene ring to which it is attached is a fused ring system represented by structure A, B or C:

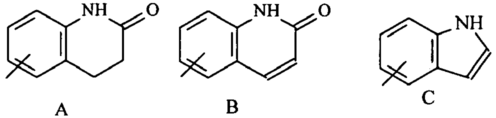

In another embodiment, the SARM compound is a compound of one of the above formulas wherein R is alkyl. In another embodiment, the SARM compound is a compound of one of the above formulas wherein R is haloalkyl. In another embodiment, the SARM compound is a compound of one of the above formulas wherein R is dihaloalkyl. In another embodiment, the SARM compound is a compound of one of the above formulas wherein R is trihaloalkyl. In another embodiment, the SARM compound is a compound of one of the above formulas wherein R is CH₂F. In another embodiment, the SARM compound is a compound of one of the above formulas wherein R is CHF₂. In another embodiment, the SARM compound is a compound of one of the above formulas wherein R is CF₃. In another embodiment, the SARM compound is a compound of one of the above formulas wherein R is CF₂CF₃. In another embodiment, the SARM compound is a compound of one of the above formulas wherein R is aryl. In another embodiment, the SARM compound is a compound of one of the above formulas wherein R is phenyl. In another embodiment, the SARM compound is a compound of one of the above formulas wherein R is F. In another embodiment, the SARM compound is a compound of one of the above formulas wherein R is I. In another embodiment, the SARM compound is a compound of one of the above formulas wherein R is Br. In another embodiment, the SARM compound is a compound of one of the above formulas wherein R is Cl. In another embodiment, the SARM compound is a compound of one of the above formulas wherein R is alkenyl. In another embodiment, the SARM compound is a compound of one of the above formulas wherein R is OH.

In another embodiment, the SARM compound is a compound of one of the above formulas wherein R₁ is CH₃. In another embodiment, the SARM compound is a compound of one of the above formulas wherein R₁ is CH₂F. In another embodiment, the SARM compound is a compound of one of the above formulas wherein R₁ is CHF₂. In another embodiment, the SARM compound is a compound of one of the above formulas wherein R₁ is CF₃. In another embodiment, the SARM compound is a compound of one of the above formulas wherein R₁ is CH₂CH₃. In another embodiment, the SARM compound is a compound of one of the above formulas wherein R₁ is CF₂CF₃.

Each substituent of each of X, Y, Z, G, T, Q, R and R₁, for each of the above formulas, represents a separate embodiment of the present invention. Further, each position enumerated above of each of the above substituents represents a separate embodiment of the present invention. Further, each number enumerated above of each of the above integers represents a separate embodiment of the present invention.

In another embodiment, the SARM compound is a compound represented by a structure of formula XIX:

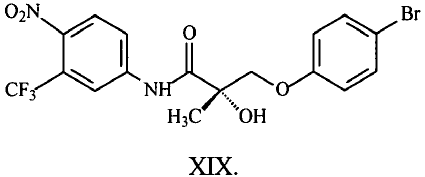

In another embodiment, the SARM compound is a compound represented by a structure of formula XX:

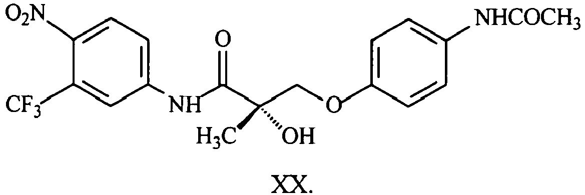

In another embodiment, the SARM compound is a compound represented by a structure of formula XXI:

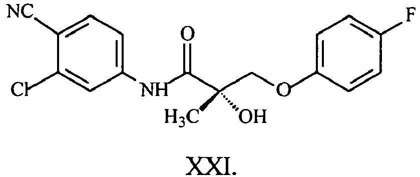

In another embodiment, the SARM compound is a compound represented by a structure of formula XXII:

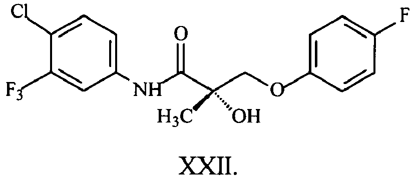

In another embodiment, the SARM compound is a compound represented by a structure of formula XXIII:

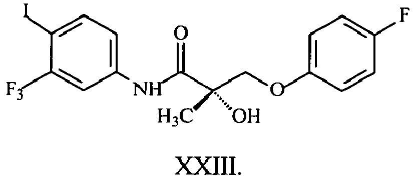

In another embodiment, the SARM compound is a compound represented by a structure of formula XXIV:

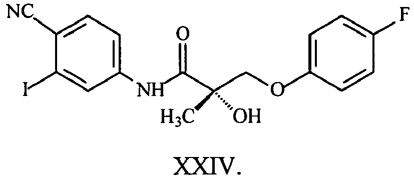

An "alkyl" group refers, in one embodiment, to a saturated aliphatic hydrocarbon, including straight chain, branched-chain and cyclic alkyl groups. In one embodiment, the alkyl group has 1-12 carbons. In another embodiment, the alkyl group has 1-7 carbons. In another embodiment, the alkyl group has 1-6 carbons. In another embodiment, the alkyl group has 1-4 carbons. The alkyl group may be unsubstituted or substituted by one or more groups selected from F, I, Br, Cl, hydroxy, alkoxy carbonyl, amido, alkylamido, dialkylamido, nitro, amino, alkylamino, dialkylamino, carboxyl, thio and thioalkyl.

An "alkenyl" group refers, in one embodiment, to an unsaturated hydrocarbon, including straight chain, branched chain and cyclic groups having one or more double bond. The alkenyl group may have one double bond, two double bonds, three double bonds etc. Examples of alkenyl groups are ethenyl, propenyl, butenyl, cyclohexenyl etc. The alkenyl group may be unsubstituted or substituted by one or more groups selected from F, I, Br, Cl, hydroxy, alkoxy carbonyl, amido, alkylamido, dialkylamido, nitro, amino, alkylamino, dialkylamino, carboxyl, thio and thioalkyl.

A "haloalkyl" group refers, in one embodiment, to an alkyl group as defined above, which is substituted by one or more halogen atoms, e.g. by F, Cl, Br or I.

An "aryl" group refers, in one embodiment, to an aromatic group having at least one carbocyclic aromatic group or heterocyclic aromatic group, which may be unsubstituted or substituted by one or more groups selected from F, I, Br, Cl, haloalkyl, hydroxy, alkoxy carbonyl, amido, alkylamido, dialkylamido, nitro, amino, alkylamino, dialkylamino, carboxy or thio or thioalkyl. Non-limiting examples of aryl rings are phenyl, naphthyl, pyranyl, pyrrolyl, pyrazinyl, pyrimidinyl, pyrazolyl, pyridinyl, furanyl, thiophenyl, thiazolyl, imidazolyl, isoxazolyl, and the like.

A "hydroxyl" group refers, in one embodiment, to an OH group. An "alkenyl" group refers to a group having at least one carbon-carbon double bond. A halo group refers, in one embodiment, to F, Cl, Br or I.

An "arylalkyl" group refers, in one embodiment, to an alkyl bound to an aryl, wherein alkyl and aryl are as defined above. An example of an arylalkyl group is a benzyl group.

### Pharmaceutical Compositions

"Pharmaceutical composition" means, in one embodiment, a therapeutically effective amount of the active ingredient, i.e. the SARM compound, together with a pharmaceutically acceptable carrier or diluent. A "therapeutically effective amount" refers, in one embodiment, to that amount which provides a therapeutic effect for a given condition and administration regimen.

The pharmaceutical compositions containing the SARM agent can be administered to a subject by any method known to a person skilled in the art, such as parenterally, paracancerally, transmucosally, transdermally, intra-muscularly, intravenously, intra-dermally, subcutaneously, intra-peritonealy, intra-ventricularly, intracranially, intra-vaginally or intra-tumorally.

In one embodiment, the pharmaceutical compositions are administered orally, and are thus formulated in a form suitable for oral administration, i.e. as a solid or a liquid preparation. Suitable solid oral formulations include tablets, capsules, pills, granules, pellets and the like. Suitable liquid oral formulations include solutions, suspensions, dispersions, emulsions, oils and the like. In one embodiment of the present invention, the SARM compounds are formulated in a capsule. In accordance with this embodiment, the compositions of the present invention comprise in addition to the SARM active compound and the inert carrier or diluent, a hard gelating capsule.

Further, in another embodiment, the pharmaceutical compositions are administered by intravenous, intra-arterial, or intra-muscular injection of a liquid preparation. Suitable liquid formulations include solutions, suspensions, dispersions, emulsions, oils and the like. In one embodiment, the pharmaceutical compositions are administered intravenously, and are thus formulated in a form suitable for intravenous administration. In another embodiment, the pharmaceutical compositions are administered intra-arterially, and are thus formulated in a form suitable for intra-arterial administration. In another embodiment, the pharmaceutical compositions are administered intra-muscularly, and are thus formulated in a form suitable for intra-muscular administration.

Further, in another embodiment, the pharmaceutical compositions are administered topically to body surfaces, and are thus formulated in a form suitable for topical administration. Suitable topical formulations include gels, ointments, creams, lotions, drops and the like. For topical administration, the SARM agents or their physiologically tolerated derivatives such as salts, esters, N-oxides, and the like are prepared and applied as solutions, suspensions, or emulsions in a physiologically acceptable diluent with or without a pharmaceutical carrier.

Further, in another embodiment, the pharmaceutical compositions are administered as a suppository, for example a rectal suppository or a urethral suppository. Further, in another embodiment, the pharmaceutical compositions are administered by subcutaneous implantation of a pellet. In a further embodiment, the pellet provides for controlled release of SARM agent over a period of time.

In another embodiment, the active compound can be delivered in a vesicle, in particular a liposome (see Langer, Science 249:1527-1533 (1990); Treat et al., in Liposomes in the Therapy of Infectious Disease and Cancer, Lopez- Berestein and Fidler (eds.), Liss, New York, pp. 353-365 (1989); Lopez-Berestein, ibid., pp. 317-327; see generally ibid).

As used herein "pharmaceutically acceptable carriers or diluents" are well known to those skilled in the art. The carrier or diluent may be a solid carrier or diluent for solid formulations, a liquid carrier or diluent for liquid formulations, or mixtures thereof.

Solid carriers/diluents include, but are not limited to, a gum, a starch (e.g. corn starch, pregeletanized starch), a sugar (e.g., lactose, mannitol, sucrose, dextrose), a cellulosic material (e.g. microcrystalline cellulose), an acrylate (e.g. polymethylacrylate), calcium carbonate, magnesium oxide, talc, or mixtures thereof.

For liquid formulations, pharmaceutically acceptable carriers may be aqueous or non-aqueous solutions, suspensions, emulsions or oils. Examples of non-aqueous solvents are propylene glycol, polyethylene glycol, and injectable organic esters such as ethyl oleate. Aqueous carriers include water, alcoholic/aqueous solutions, emulsions or suspensions, including saline and buffered media. Examples of oils are those of petroleum, animal, vegetable, or synthetic origin, for example, peanut oil, soybean oil, mineral oil, olive oil, sunflower oil, and fish-liver oil.

Parenteral vehicles (for subcutaneous, intravenous, intra-arterial, or intra-muscular injection) include sodium chloride solution, Ringer's dextrose, dextrose and sodium chloride, lactated Ringer's and fixed oils. Intravenous vehicles include fluid and nutrient replenishers, electrolyte replenishers such as those based on Ringer's dextrose, and the like. Examples are sterile liquids such as water and oils, with or without the addition of a surfactant and other pharmaceutically acceptable adjuvants. In general, water, saline, aqueous dextrose and related sugar solutions, and glycols such as propylene glycols or polyethylene glycol are preferred liquid carriers, particularly for injectable solutions. Examples of oils are those of petroleum, animal, vegetable, or synthetic origin, for example, peanut oil, soybean oil, mineral oil, olive oil, sunflower oil, and fish-liver oil.

In addition, the compositions may further comprise binders (e.g. acacia, cornstarch, gelatin, carbomer, ethyl cellulose, guar gum, hydroxypropyl cellulose, hydroxypropyl methyl cellulose, povidone), disintegrating agents (e.g. cornstarch, potato starch, alginic acid, silicon dioxide, croscarmelose sodium, crospovidone, guar gum, sodium starch glycolate), buffers (e.g., Tris-HCI., acetate, phosphate) of various pH and ionic strength, additives such as albumin or gelatin to prevent absorption to surfaces, detergents (e.g., Tween 20, Tween 80, Pluronic F68, bile acid salts), protease inhibitors, surfactants (e.g. sodium lauryl sulfate), permeation enhancers, solubilizing agents (e.g., glycerol, polyethylene glycerol), anti-oxidants (e.g., ascorbic acid, sodium metabisulfite, butylated hydroxyanisole), stabilizers (e.g. hydroxypropyl cellulose, hyroxypropylmethyl cellulose), viscosity increasing agents (e.g. carbomer, colloidal silicon dioxide, ethyl cellulose, guar gum), sweetners (e.g. aspartame, citric acid), preservatives (e.g., Thimerosal, benzyl alcohol, parabens), lubricants (e.g. stearic acid, magnesium stearate, polyethylene glycol, sodium lauryl sulfate), flow-aids (e.g. colloidal silicon dioxide), plasticizers (e.g. diethyl phthalate, triethyl citrate), emulsifiers (e.g. carbomer, hydroxypropyl cellulose, sodium lauryl sulfate), polymer coatings (e.g., poloxamers or poloxamines), coating and film forming agents (e.g. ethyl cellulose, acrylates, polymethacrylates) and/or adjuvants.

In one embodiment, the pharmaceutical compositions provided herein are controlled release compositions, i.e. compositions in which the SARM compound is released over a period of time after administration. Controlled or sustained release compositions include formulation in lipophilic depots (e.g. fatty acids, waxes, oils). In another embodiment, the composition is an immediate release composition, i.e. a composition in which all of the SARM compound is released immediately after administration.

In yet another embodiment, the pharmaceutical composition can be delivered in a controlled release system. For example, the agent may be administered using intravenous infusion, an implantable osmotic pump, a transdermal patch, liposomes, or other modes of administration. In one embodiment, a pump may be used (see Langer, supra; Sefton, CRC Crit. Ref. Biomed. Eng. 14:201 (1987); Buchwald et al., Surgery 88:507 (1980); Saudek et al., N. Engl. J. Med. 321:574 (1989). In another embodiment, polymeric materials can be used. In yet another embodiment, a controlled release system can be placed in proximity to the therapeutic target, i.e., the brain, thus requiring only a fraction of the systemic dose (see, e.g., Goodson, in Medical Applications of Controlled Release, supra, vol. 2, pp. 115-138 (1984). Other controlled release systems are discussed in the review by Langer (Science 249:1527-1533 (1990).

The compositions may also include incorporation of the active material into or onto particulate preparations of polymeric compounds such as polylactic acid, polglycolic acid, hydrogels, etc, or onto liposomes, micro-emulsions, micelles, unilamellar or multilamellar vesicles, erythrocyte ghosts, or spheroplasts.) Such compositions will influence the physical state, solubility, stability, rate of *in vivo* release, and rate of in *vivo* clearance.

Also comprehended by the invention are particulate compositions coated with polymers (e.g. poloxamers or poloxamines) and the compound coupled to antibodies directed against tissue-specific receptors, ligands or antigens or coupled to ligands of tissue-specific receptors.

Also comprehended by the invention are compounds modified by the covalent attachment of water-soluble polymers such as polyethylene glycol, copolymers of polyethylene glycol and polypropylene glycol, carboxymethyl cellulose, dextran, polyvinyl alcohol, polyvinylpyrrolidone or polyproline. The modified compounds are known to exhibit substantially longer half-lives in blood following intravenous injection than do the corresponding unmodified compounds (Abuchowski et al., 1981; Newmark et al., 1982; and Katre et al., 1987). Such modifications may also increase the compound's solubility in aqueous solution, eliminate aggregation, enhance the physical and chemical stability of the compound, and greatly reduce the immunogenicity and reactivity of the compound. As a result, the desired *in vivo* biological activity may be achieved by the administration of such polymer-compound abducts less frequently or in lower doses than with the unmodified compound.

The preparation of pharmaceutical compositions that contain an active component is well understood in the art, for example by mixing, granulating, or tablet-forming processes. The active therapeutic ingredient is often mixed with excipients that are pharmaceutically acceptable and compatible with the active ingredient. For oral administration, the SARM agents or their physiologically tolerated derivatives such as salts, esters, N-oxides, and the like are mixed with additives customary for this purpose, such as vehicles, stabilizers, or inert diluents, and converted by customary methods into suitable forms for administration, such as tablets, coated tablets, hard or soft gelatin capsules, aqueous, alcoholic or oily solutions. For parenteral administration, the SARM agents or their physiologically tolerated derivatives such as salts, esters, N-oxides, and the like are converted into a solution, suspension, or emulsion, if desired with the substances customary and suitable for this purpose, for example, solubilizers or other.

An active component can be formulated into the composition as neutralized pharmaceutically acceptable salt forms. Pharmaceutically acceptable salts include the acid addition salts (formed with the free amino groups of the polypeptide or antibody molecule), which are formed with inorganic acids such as, for example, hydrochloric or phosphoric acids, or such organic acids as acetic, oxalic, tartaric, mandelic, and the like. Salts formed from the free carboxyl groups can also be derived from inorganic bases such as, for example, sodium, potassium, ammonium, calcium, or ferric hydroxides, and such organic bases as isopropylamine, trimethylamine, 2-ethylamino ethanol, histidine, procaine, and the like.

For use in medicine, the salts of the SARM will be pharmaceutically acceptable salts. Other salts may, however, be useful in the preparation of the compounds according to the invention or of their pharmaceutically acceptable salts. Suitable pharmaceutically acceptable salts of the compounds of this invention include acid addition salts which may, for example, be formed by mixing a solution of the compound according to the invention with a solution of a pharmaceutically acceptable acid such as hydrochloric acid, sulphuric acid, methanesulphonic acid, fumaric acid, maleic acid, succinic acid, acetic acid, benzoic: acid, oxalic acid, citric acid, tartaric acid, carbonic acid or phosphoric acid.

As used herein, the term "administering" refers to bringing a subject in contact with a SARM compound of the present invention. As used herein, administration can be accomplished *in vitro,* i.e. in a test tube, or *in vivo,* i.e. in cells or tissues of living organisms, for example humans. In one embodiment, the present invention encompasses administering the compounds of the present invention to a subject.

In another embodiment, the term "contacting" means that the SARM compound of the present invention is introduced into a subject receiving treatment, and the SARM compound is allowed to come in contact with the AR in vivo.

In one embodiment, the methods of the present invention comprise administering a SARM compound as the sole active ingredient. However, also encompassed within the scope of the present invention are methods for treating and/or preventing bone-related disorders, which comprise administering the SARM compounds in combination with one or more therapeutic agents. These agents include, but are not limited to: LHRH analogs, reversible anti-androgens, anti-estrogens, anticancer drugs, 5-alpha reductase inhibitors, aromatase inhibitors, progestins, agents acting through other nuclear hormone receptors, selective estrogen receptor modulators (SERM), progesterone, estrogen, PDE5 inhibitors, apomorphine, bisphosphonate, and one or more additional SARMS.

Thus, in one embodiment, the methods of the present invention comprise administering the SARM compound in combination with an LHRH analog. In another embodiment, the methods of the present invention comprise administering a SARM compound in combination with a reversible anti-androgen. In another embodiment, the methods of the present invention comprise administering a SARM compound in combination with an anti-estrogen. In another embodiment, the methods of the present invention comprise administering a SARM compound in combination with an anticancer drug. In another embodiment, the methods of the present invention comprise administering a SARM compound in combination with a 5-alpha reductase inhibitor. In another embodiment, the methods of the present invention comprise administering a SARM compound in combination with an aromatase inhibitor. In another embodiment, the methods of the present invention comprise administering a SARM compound in combination with a progestin. In another embodiment, the methods of the present invention comprise administering a SARM compound in combination with an agent acting through other nuclear hormone receptors. In another embodiment, the methods of the present invention comprise administering a SARM compound in combination with a selective estrogen receptor modulator (SERM). In another embodiment, the methods of the present invention comprise administering a SARM compound in combination with a progesterone. In another embodiment, the methods of the present invention comprise administering a SARM compound in combination with an estrogen. In another embodiment, the methods of the present invention comprise administering a SARM compound in combination with a PDE5 inhibitor. In another embodiment, the methods of the present invention comprise administering a SARM compound in combination with apomorphine. In another embodiment, the methods of the present invention comprise administering a SARM compound in combination with a bisphosphonate. In another embodiment, the methods of the present invention comprise administering a SARM compound, in combination with one or more additional SARMS.

### EXAMPLES

### EXAMPLE 1

### EXPERIMENTAL DESIGN- EXAMPLES 1-14

Animals were randomized (n=10 per group) into each of the treatment groups outlined in the table below. Animals assigned to some groups underwent surgical ovariectomy (OVX) on day 1 of the experiment. Drug administration with Compound VI, Compound IX and Compound XI, anti-androgen, and/or DHT began immediately (i.e., on the day that OVX was performed) or 90 days after OVX to assess the ability of these compounds to inhibit bone resorption (immediate treatment) or stimulate bone formation (delayed treatment). The compound of interest was administered via daily subcutaneous injection (0.25 milliliter [mL]) until day 180 of the study. Drug solutions were prepared daily by dissolving in ethanol and dilution with polyethylene glycol 300. The percentage of ethanol was the same in all vehicles, and was determined based on the solubility of the test compounds.

Whole body dual energy x-ray absorptiometry (DEXA) images were collected for up to 210 days after OVX, as described in the table below. BMD, BMC, bone mineral area (BMA), lean body mass (LBM), FM, total body mass (TBM), and sub-regional BMD in the lumbar vertebrae and left femur were determined at each time point.

All animals were sacrificed 120 days following initiation of treatment. Femurs and tibias were excised from the sacrificed rats for future studies. Serum and urine specimens were collected prior to or at the time of sacrifice and used to determine serum concentrations of osteocalcin, IL-6, IGF-1, and urinary concentrations of deoxypyridinoline, and creatinine for animals in each group.

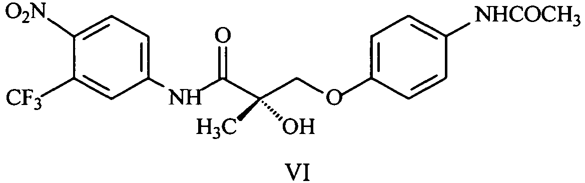

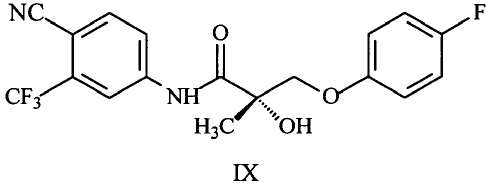

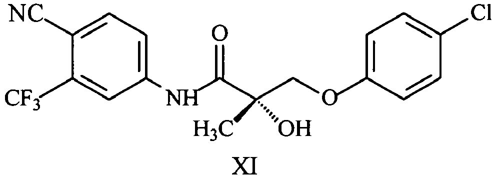

**Table 1: Experimental groups for Examples 1-14**

| **Group #** | **Gonadal status** | **Treatment** | **Days on which DEXA was performed** |
|---|---|---|---|
| 1 | Intact | -- | 0, 30, 60, 90, 120, 150, 180,210 |
| 2 | Intact | 1.0 mg/day DHT IMMEDIATE | 0,30,60,90 |
| 3 | Intact | 1.0 mg/day VI IMMEDIATE | |
| 4 | OVX | -- | 0,30,60,90 |
| 5 | OVX | 1.0 mg/day DHT IMMEDIATE | 0,30,60,90 |
| 6 | OVX | 1.0 mg/day VI + 0.5 mg/day bicalutamide IMMEDIATE | 0,30,60,90 |
| 7 | OVX | 0.1 mg/day VI IMMEDIATE | 0, 30, 60, 90 |
| 8 | OVX | 0.3 mg/day VI IMMEDIATE | 0, 30, 60, 90 |
| 9 | OVX | 0.5 mg/day VI IMMEDIATE | 0, 30, 60, 90 |
| 10 | OVX | 0.75 mg/day VI IMMEDIATE | 0,30,60,90 |
| 11 | OVX | 1.0 mg/day VI IMMEDIATE | 0,30,60,90 |
| 12 | OVX | 3.0 mg/day VI IMMEDIATE | 0, 30, 60, 90 |
| 13 | Intact | 1.0 mg/day DHT DELAYED | 0, 90, 120, 150, 180, 210 |
| 14 | Intact | 1.0 mg/day VI DELAYED | 0, 90, 120, 150, 180, 210 |
| 15 | OVX | -- | 0, 90, 120, 150, 180, 210 |
| 16 | OVX | 1.0 mg/day DHT DELAYED | 0, 90, 120, 150, 180, 210 |
| 17 | OVX | 1.0 mg/day VI + 0.5 mg/day bicalutamide DELAYED | 0, 90, 120, 150, 180, 210 |
| 18 | OVX | 0.1 mg/day VI DELAYED | 0, 90, 120, 150, 180, 210 |
| 19 | OVX | 0.3 mg/day VI DELAYED | 0, 90, 120, 150, 180, 210 |
| 20 | OVX | 0.5 mg/day VI DELAYED | 0, 90, 120, 150, 180, 210 |
| 21 | OVX | 0.75 mg/day VI DELAYED | 0, 90, 120, 150, 180, 210 |
| 22 | OVX | .0 mg/day VI DELAYED | 0, 90, 120, 150, 180, 210 |
| 23 | OVX | 3.0 mg/day VI DELAYED | 0, 90, 120, 150, 180, 210 |
| 24 | OVX | 1.0 mg/day IX IMMEDIATE | 0, 90, 120, 150, 180, 210 |
| 25 | OVX | 1.0 mg/day XI IMMEDIATE | 0, 90, 120, 150, 180, 210 |
| 26 | OVX | 1.0 mg/day IX DELAYED | 0, 90, 120, 150, 180, 210 |
| 27 | OVX | 1.0 mg/day XI DELAYED | 0, 90, 120, 150, 180, 210 |

### EXAMPLE 2

### COMPOUND VI PREVENTS LOSS OF BMD IN A RAT OSTEOPOROSIS

### MODEL

### MATERIALS AND EXPERIMENTAL METHODS (EXAMPLES 2-14) ANIMALS

Female Sprague-Dawley rats were purchased from Harlan (Indianapolis, IN). The animals were housed three per cage, were allowed free access to tap water and commercial rat chow (Harlan Teklad 22/5 rodent diet - 8640), and were maintained on a 12 hr light:dark cycle. This study was reviewed and approved by the Institutional Laboratory Care and Use Committee of The Ohio State University.

### EXPERIMENTAL DESIGN

At 23 weeks of age, the animals were ovariectomized (OVX) or sham-operated and then assigned to one of 12 treatment groups (Table 2) of 10 animals/group, receiving various amounts of Compound VI, other treatments, or no treatment, as described in the Results section. Sham-operated animals are referred to herein as "intact," to indicate that the ovaries have not been removed. During the course of the study, five animals died from non-drug related causes. Therefore, groups 1, 6, and 10 were composed of nine animals each, and group 4 was composed of eight animals. Dosing solutions were prepared daily by dissolving drug in DMSO and diluting in polyethylene glycol 300 (PEG 300). All doses were administered for 120 days via daily subcutaneous injections in a volume of 0.20 ml.

**Table 2: Experimental groups for Examples 2-8.**

| **Group Number** | **Gonadal Status** | **Compound VI (mg/day)** | **DHT (mg/day)** | **Bicalutamide (mg/day)** |
|---|---|---|---|---|
| 1 | Intact | -- | -- | -- |
| 2 | Intact | -- | 1.0 | -- |
| 3 | Intact | 1.0 | -- | -- |
| 4 | OVX | -- | -- | -- |
| 5 | OVX | -- | 1.0 | -- |
| 6 | OVX | 0.5 | -- | 1.0 |
| 7 | OVX | 0.1 | -- | -- |
| 8 | OVX | 0.3 | -- | -- |
| 9 | OVX | 0.5 | -- | -- |
| 10 | OVX | 0.75 | -- | -- |
| 11 | OVX | 1.0 | -- | -- |
| 12 | OVX | 3.0 | -- | -- |

Immediately following the whole body DEXA scan on day 120, groups 2 through 12 were sacrificed, and the lumbar vertebra, femurs, and tibia were excised and cleared of soft tissue. The intact control group for this study (Group 1) also served as a control group for the concurrent delayed treatment study described in Examples 9-13. Therefore, Group 1 was sacrificed at day 210.

### Body parameter measurements

Total body BMD, percent FM, body weight (BW), BMC, bone mineral area (BMA), and lean mass (LM) were determined by DEXA (GE, Lunar Prodigy™) using the small animal software (Lunar enCORE, version 6.60.041) on days 0 and 120. Animal body weight was also determined by standard gravimetric methods using a 700 series Ohaus triple beam animal balance (Florham Park, NJ). For DEXA scanning, the animals were anesthetized with ketamine:xylazine (87:13 mg/kg) and positioned in a prone position. Total body data was obtained by selecting an area encompassing the entire animal as the region of interest during data processing. The parameters determined to be the most sensitive to estrogen withdrawal (i.e., largest differences between intact and OVX control groups) were used and reported herein in order to focus our analyses on the most hormone-sensitive measures with a larger dynamic range.

Excised bones were scanned through a 3-inch deep room temperature water bath to simulate soft tissue. The proximal femur, distal femur, proximal tibia, L2-L4 vertebra, and L5-L6 vertebrae were selected as regions of interest from the DEXA scan and analyzed for BMD. Femoral images were also subdivided into ten equal regions of interest from proximal (region 1) to distal (region 10), and the BMD of each region was determined by the Lunar enCORE small animal software.

Right femurs from the OVX + 1.0 mg/day Compound VI (Group 11), OVX + 3.0 mg/day Compound VI (Group 12), OVX + 1.0 mg/day DHT (Group 5), OVX control (Group 4), intact + 1 mg/day Compound VI (Group 3), and intact control (Group 1) were sent to Skeletech, Inc. (Bothell, WA) for peripheral quantitative computed tomography (pQCT) analysis and biomechanical testing. Femurs were subjected to pQCT scanning using a Stratec XCT RM and associated software (Stratec Medizintechnik GmbH, Pforzheim, Germany. Software version 5.40 C). Femurs were analyzed at both the mid-shaft and distal regions. Lengths of femurs were determined using scout scan views, and the mid-shaft region (50% of the length of the femur) and the distal region (20% of the length of the femur starting at the distal end) were selected as regions of interest. One 0.5 mm slice perpendicular to the long axis of the femur was used for analysis. Total BMC, total bone area, total BMD, cortical bone mineral content, cortical bone area, cortical BMD, cortical thickness, periosteal perimeter (circumference) and endosteal perimeter were determined at the mid-shaft of the femur. At the distal femur, total BMC, total bone area, total BMD, trabecular bone mineral content, trabecular bone area and trabecular BMD were determined.

After pQCT analysis, de-fleshed whole femurs were used in the three-point bending test. The anterior to posterior diameter (APD) (unit: millimeter [mm]) at the midpoint of the femoral shaft was measured with an electronic caliper. The femur was placed on the lower supports of a three-point bending fixture with the anterior side of the femur facing downward in an Instron Mechanical Testing Machine (Instron 4465 retrofitted to 5500)(Canton, MA). The length (L) between the lower supports was set to 14 mm. The upper loading device was aligned to the center of the femoral shaft. The load was applied at a constant displacement rate of 6 mm/min until the femur broke. The mechanical testing machine directly measured the maximum load (Fu) (unit:N), stiffness (S) (units:N/mm), and energy absorbed (W) (unit:mJ). The axial area moment of inertia (I) (unit:mm4) was calculated by the software during the pQCT analysis of the femoral mid-shaft. Stress (σ) (units:N/mm2), elastic modulus (E) (unit:Mpa), and toughness (T) (units:mJ/m3) were calculated by the following formulas: stress: σ = (Fu * L *(a/2)) / (4* I); elastic modulus: E = S*L3/(48*I); and toughness: T = 3*W*(APD/2)2/(L*I).

### Statistical analyses

Statistical analyses were performed by single factor analysis of variance (ANOVA). P-values of less than 0.05 were considered statistically significant differences.

### RESULTS

Rats were assigned to one of 12 treatment groups. Groups 4-12 were ovariectomized on day 0 of the study, while groups 1-3 were intact rats. Groups 7-12 received Compound VI by daily subcutaneous injection at doses of 0.1, 0.3, 0.5, 0.75, 1.0, and 3 mg/day, respectively. Groups 1 and 4 were intact (i.e, non-OVX) and OVX negative control groups, respectively, receiving DMSO alone. Groups 2 and 5 (intact and OVX) received the androgen dihydrotestosterone (DHT) (1 mg/day) as a positive control. Group 3 were intact rats receiving 1.0 mg/day Compound VI. Group 6 (OVX) received 0.5 mg/day of Compound VI and 1.0 mg/day of the anti-androgen bicalutamide, in order to delineate the AR-mediated versus AR-independent effects of Compound VI. BMC was determined on days 1, 30, 60, 90, and 120.

Figure 1 depicts the whole body BMD for all groups at day 120. As expected, the BMD in OVX rats (0.196 g/cm²) was significantly less than that observed in intact controls (0.214 g/cm²) at day 120. Compound VI treatment either partially (i.e., BMD significantly greater than OVX controls) or fully (i.e., BMD not significantly different than intact controls) prevented the loss of skeletal BMD in OVX rats at doses greater than 0.1 mg/day. DHT fully maintained BMD in the OVX rats. However, in intact rats, DHT caused a significant decrease in BMD, while Compound VI treatment in intact rats maintained BMD at the level of intact controls. Co-administration of the anti-androgen bicalutamide partially prevented the effects of Compound VI, showing that the AR partially mediated the bone response to Compound VI. Thus, Compound VI prevented loss of BMD in OVX rats.

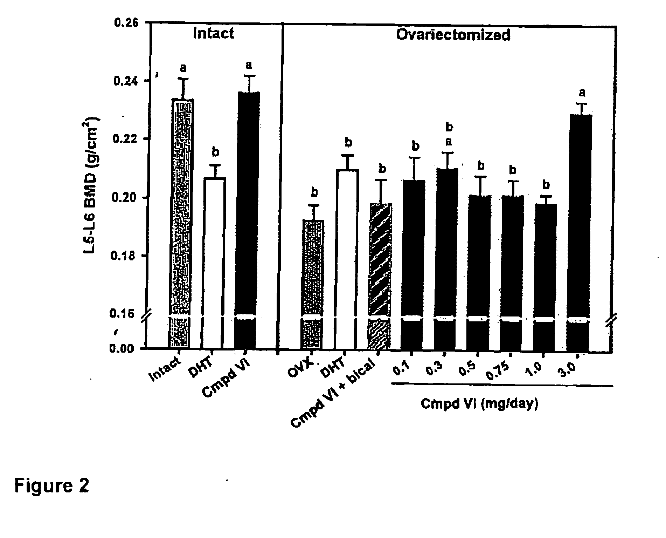

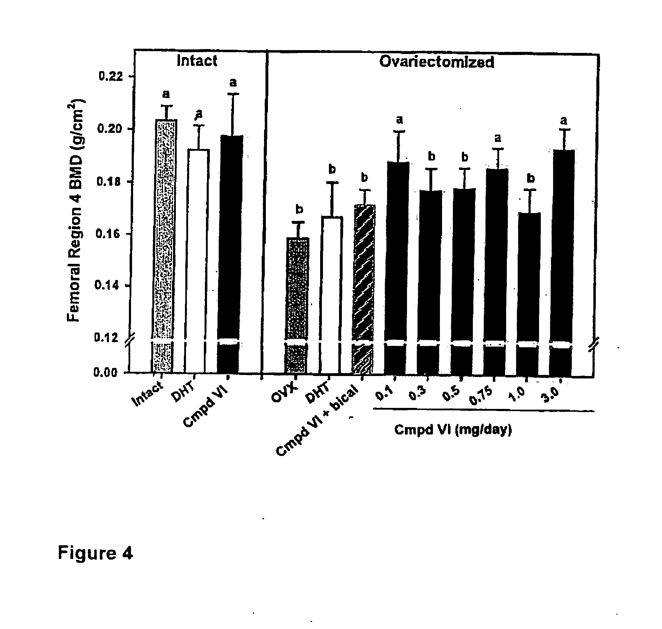

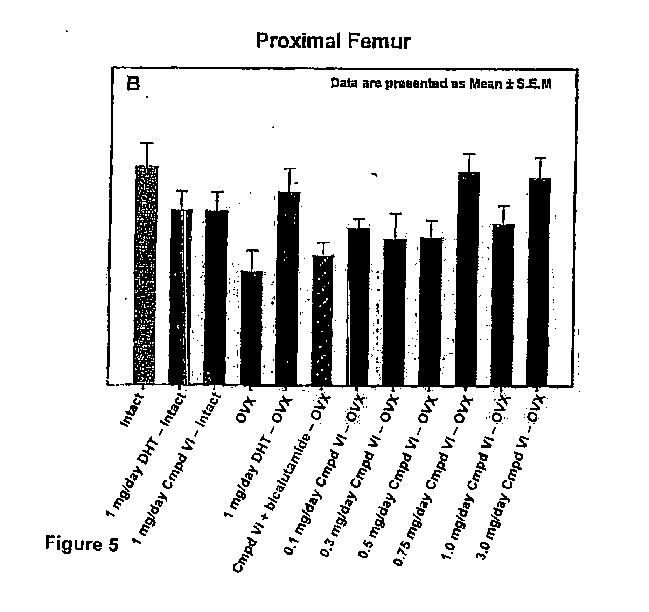

Figure 2 depicts results of DEXA analysis of excised L5-L6 vertebrae. While control OVX rats lost a significant amount of vertebral BMD over the course of the study, Compound VI treatment had a dose-dependent bone-sparing effect, with 3 mg/day Compound VI completely preventing, and 0.5 and 1 mg/day Compound VI partially preventing, OVX-induced bone loss. OVX rats administered 0.1,0.3, and 0.75 mg/day of Compound VI exhibited higher BMD than control OVX rats, but the difference was not statistically significant. Co-administration of bicalutamide partially prevented the bone-sparing effect of Compound VI. In contrast to Compound VI, DHT treatment in OVX rats did not prevent bone loss in the L5-L6 vertebrae. Compound VI had no effect on BMD in intact rats, while DHT treatment significantly decreased BMD to a level similar to OVX controls. Compound VI prevented OVX-induced BMD decreases in L2-L4 vertebrae (Figure 3), region 4 of the femur (Figure 4), and the proximal femur (Figure 5) as well. Thus, Compound VI prevented OVX-induced BMD decreases in the L2-L4 and L5-L6 vertebrae.

The findings in this Example show that Compound VI prevents loss in BMD due to ovariectomy, both globally and in several specific locations in the body. Thus, SARMS are useful in preventing bone loss due to hormonal causes such as menopause.

### EXAMPLE 3

### COMPOUND VI PREVENTS LOSS OF CORTICAL BONE DUE TO OSTEOPOROSIS AND INCREASES CORTICAL BONE MASS IN HEALTHY SUBJECTS

Cortical thickness (CT) at the femoral mid-shaft of the rats from Example 2 was determined (Figure 6). OVX rats exhibited decreased cortical density relative to intact control rats. While Compound VI and DHT both prevented the decrease in CT, Compound VI-treated groups exhibited a higher CT than DHT treated groups. Additionally, intact rats and OVX rats receiving Compound VI showed significant increases in CT above the level of intact controls.

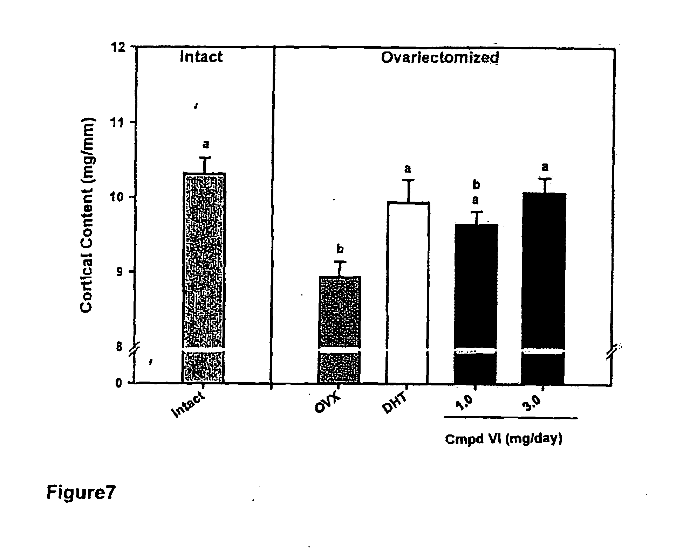

Cortical content (CC) at the mid-shaft of the femur was also assessed (Figure 7). A significant loss in CC from 10.3 to 8.8 mg/mm was observed in OVX control rats. Compound VI completely blocked the loss in CC, while the loss was only partially prevented by DHT. In addition, the group receiving 3 mg/day of Compound VI exhibited an increase in CC over intact control levels.

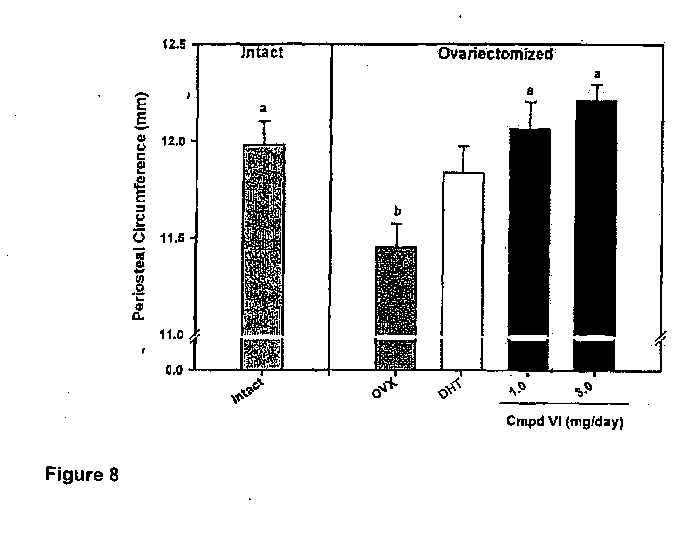

Periosteal circumference (PC) of the femoral mid-shaft was also measured (Figure 8). While PC was decreased in OVX rats, the decrease was completely prevented by Compound VI treatment.

Cortical bone mineral density (CD) of the femoral mid-shaft was measured by pQCT. Compound VI completely prevented the loss in CD caused by OVX, while DHT only partially prevented the loss in CD. Intact rats receiving Compound VI showed an increase in CD compared to OVX and intact control rats.

CT, CC, PC, and CD are indicators of cortical bone content, density, and strength. Thus, the finding that Compound VI stabilizes these indicators in OVX rats shows that the bone-stabilizing quality of SARMS is manifest in cortical bone. Additionally, the findings of this Example show that SARMS increase cortical bone in both osteoporotic (OVX) and non-osteoporotic subjects.

### EXAMPLE 4

### COMPOUND VI PREVENTS LOSS OF TRABECULAR BONE DUE TO OSTEOPOROSIS AND INCREASES TRABECULAR BONE MASS IN HEALTHY SUBJECTS

Trabecular BMD was measured at the distal femur of the rats from Example 2 (Figure 9). Significant trabecular bone loss, from 735 to 609 mg/cm³, was observed following OVX, which was partially prevented by Compound VI and DHT. Additionally, Compound VI treatment in intact rats resulted in an increase of trabecular BMD to a level significantly higher than intact controls.

The findings of this Example indicate that the bone-stabilizing quality of SARMS is manifest in trabecular bone. Additionally, the findings show that SARMS increase trabecular bone in both osteoporotic (OVX) and non-osteoporotic subjects.

### EXAMPLE 5

### COMPOUND VI STRENGTHENS BONE IN BOTH OSTEOPOROTIC AND HEALTHY SUBJECTS

Biomechanical strength of the femurs was determined as well (Figure 10). OVX control rats exhibited a significant drop in femoral biomechanical strength, which was completely prevented by Compound VI treatment and DHT treatment. Compound VI showed no effect on intact rats.

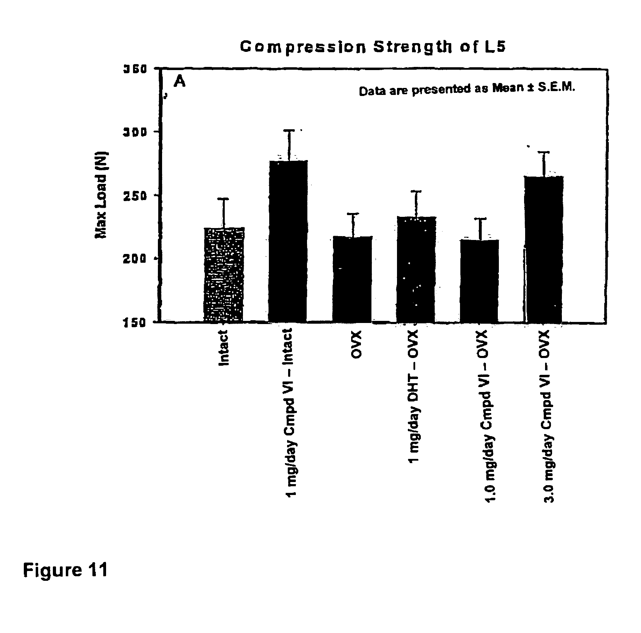

In addition, compression strength (CS) of the rats' bone was measured, in this case of the L5 vertebra (Figure 11). While OVX did not result in a significant drop in CS, Compound VI increased CS in both intact and OVX rats.

The findings of this Example indicate that SARMS strengthen bone in both osteoporotic (OVX) and non-osteoporotic subjects.

### EXAMPLE 6

### COMPOUND VI INCREASES BMC IN OSTEOPOROTIC SUBJECTS

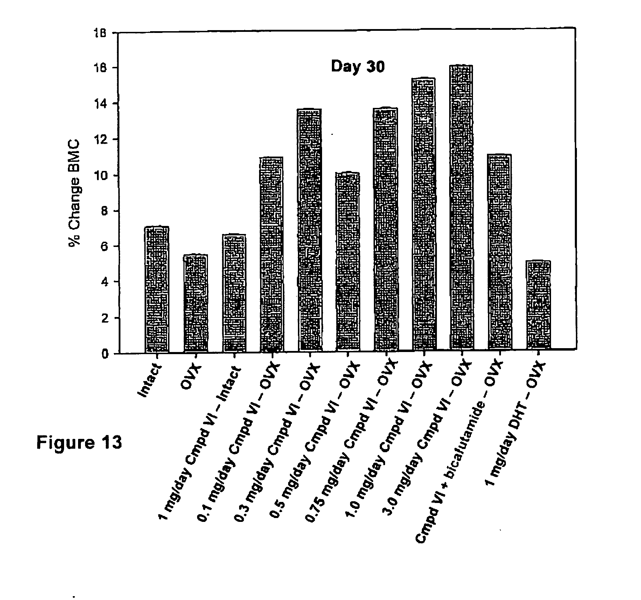

A time and dose-dependent increase in BMC was observed for all Compound VI-treated groups of the experiment described in Example 2, with increases of 22.9, 26.0, 28.5, 30.5, 30.0, and 40.1 % in groups 7-12 at 120 days, respectively, relative to control OVX rats (Figure 12A-B). DHT increased BMC by a lesser amount (15%). At the 30-day time point, Compound VI-treated mice, but not DHT-treated mice, exhibited increases in BMC (Figure 13). Thus, Compound VI increased BMC in OVX rats, demonstrating that SARMS improve BMC in osteoporotic subjects.

### EXAMPLE 7

### COMPOUND VI DECREASES FAT MASS AND INCREASES LEAN MASS BMC IN OSTEOPOROTIC SUBJECTS

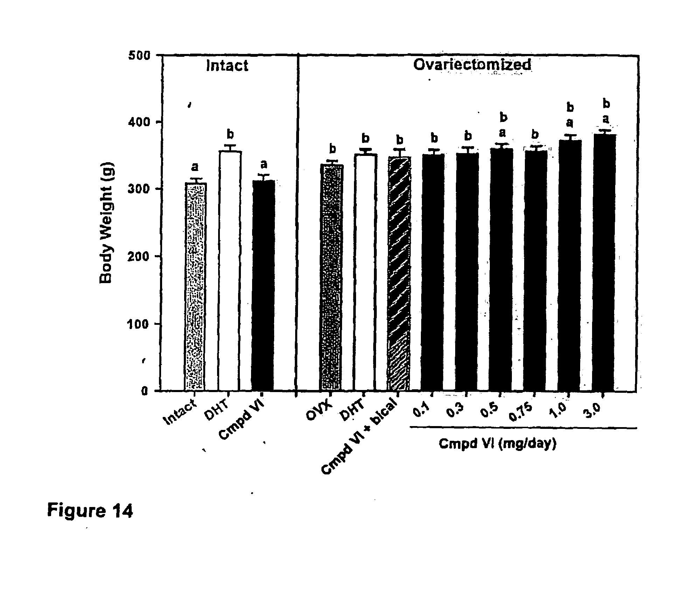

The average body weight for all groups at the beginning of the study was 267 ± 17 g (Mean ± S.D., n = 120). All rats gained a significant amount of weight over the course of the study (Figure 14). Body weight was greater in all OVX groups than in the intact control group, indicating influence of estrogen-deprivation on rat growth. A further increase in body weight was observed for the 3 mg/day Compound VI group. In intact rats, DHT resulted in an increase in body weight relative to intact controls, while Compound VI resulted in a significant decrease relative to both OVX and intact controls.

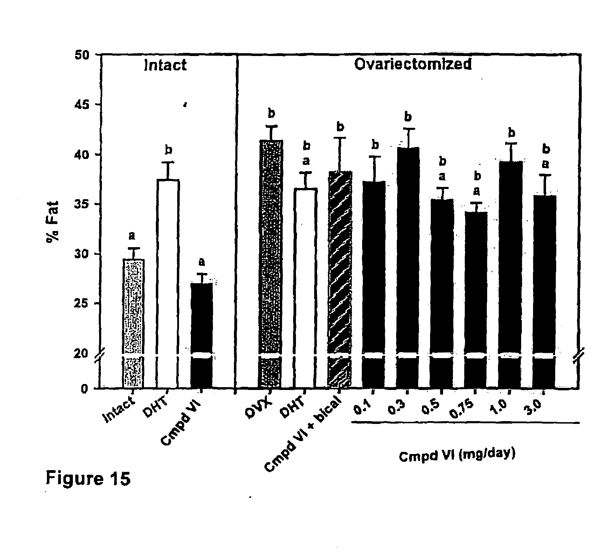

Percent fat mass (FM) at day 120 was measured by DEXA (Figure 15). The OVX control group exhibited a significantly higher FM than intact controls, illustrating the effect of estrogen deprivation on body composition. Compound VI treatment decreased FM in a dose-dependent manner, with FM levels equal to the intact control levels in the 3 mg/day group; the Compound VI-mediated decrease was prevented by co-administration of bicalutamide. DHT treatment in both intact and OVX rats increased FM to values higher than intact controls but lower than those observed in OVX controls. Intact rats receiving Compound VI exhibited a decrease in FM compared to intact controls. Corresponding changes in percentage lean mass were observed in all groups. Thus, Compound VI prevented OVX-induced increases in percent FM.

The findings of this Example show that SARMS can prevent an increase in the lean mass/FM ratio in osteoporotic subjects.

### EXAMPLE 8

### COMPOUND VI PREVENTS A RISE IN SERUM OSTEOCALCIN IN OSTEOPOROTIC SUBJECTS

Osteocalcin was measured in serum samples drawn immediately prior to sacrifice. OVX increased osteocalcin levels, and treatment with both Compound VI and DHT returned the levels to that observed in non-OVX controls (Figure 16).

In conclusion, Examples 2-8 show that Compound VI inhibited loss of both cortical and trabecular bone, loss of bone strength, and increase in FM in osteoporotic subjects. Moreover, Compound VI exhibited many of these properties in non-osteoporotic subjects as well. Further, in most cases the positive effect of Compound VI was comparable to or greater than DHT. Thus, the present invention demonstrates that (a) SARMS have osteo-anabolic effects in both the presence and absence of osteoporosis and that (b) SARMS have anti-resorptive effects that combat the results of osteoporosis.

### EXAMPLE 9

### COMPOUND VI REVERSES LOSS OF BMD IN OSTEOPOROTIC SUBJECTS

### MATERIALS AND EXPERIMENTAL METHODS

Mice in Examples 9-13 were ovariectomized and subjected to the same treatments described in Example 2, in this case, however, the treatments were not initiated until day 90 after OVX. Mice were sacrificed at day 210 and analyzed as described in Example 2.

### RESULTS

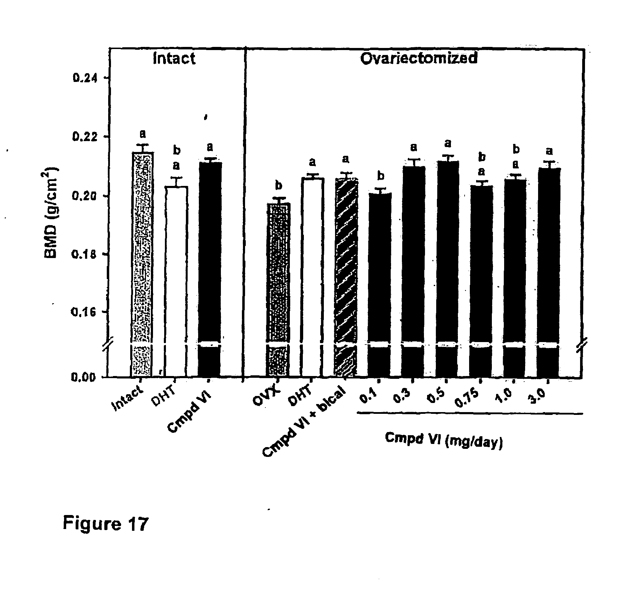

The OVX control group had a lower whole body BMD (0.197 g/cm²) than the intact control group (0.212 g/cm²), as depicted in Figure 17. Compound VI significantly reversed the decline in BMD in the 0.3, 0.5, 0.75, 1.0, and 3.0 mg/day dose groups to 0.204, 0.209, 0.206, 0.205, 0.205, and 0.206 g/cm², respectively. By contrast, DHT did not restore BMD. Neither DHT nor Compound VI increased BMD in intact animals. Compound VI increased BMD in intact controls by a non-statistically significant amount to 0.214 g/cm²; by contrast, DHT decreased BMD to 0.205 g/cm². Animals receiving co-administration of Compound VI and bicalutamide with did not differ from animals receiving Compound VI alone. Thus, Compound VI reversed the decline in BMD in osteoporotic rats.

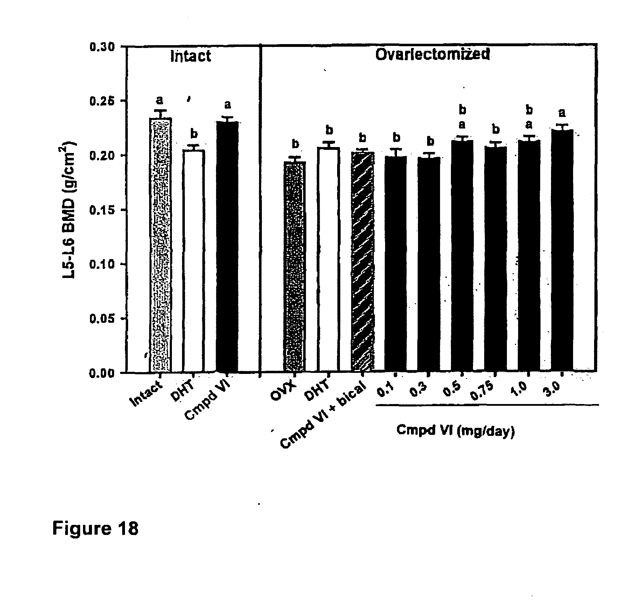

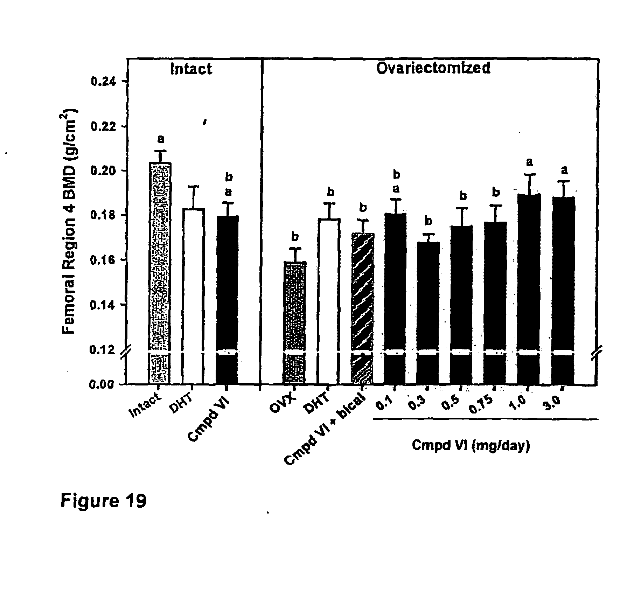

As with whole body BMD, OVX negatively affected the BMD in the L5-L6 vertebra, causing a decrease from 0.234 g/cm² in intact animals to 0.192 g/cm² in OVX controls (Figure 18). L5-L6 BMD was completely restored or significantly increased relative to control OVX animals in groups receiving 3.0 mg/day and 0.3 mg/day, respectively; other dosages of Compound VI caused increases that did not reach statistical significance. Similarly, DHT treatment partially restored the L5-L6 BMD in OVX animals. Compound VI did not affect L5-L6 BMD in intact animals; while DHT resulted in a significant decrease to a level similar to OVX controls. L5-L6 BMD in animals treated with Compound VI + bicalutamide was not significantly different from that observed in animals treated with the same amount of Compound VI alone. Similar results were observed with femoral BMD measurements (Figure 19), except that in this case, statistical significance was reached at the 0.1, 0.75, and 3.0 mg/day dosages of Compound VI.

Thus, Compound VI restores BMD lost as a result of OVX. The results of this Example demonstrate that SARMS can reverse BMD loss resulting from osteoporosis. Delaying treatment until after osteoporosis had occurred allowed assessment of anabolic activity of Compound VI, in a setting wherein anti-resorptive activity should be less of a contributor. Thus, osteo-anabolic activity is at least one of the mechanisms by which SARMS increase bone mass in osteoporotic and non-osteoporotic subjects.

### EXAMPLE 10

### COMPOUND VI REVERSES LOSS OF CORTICAL BONE IN OSTEOPOROTIC SUBJECTS

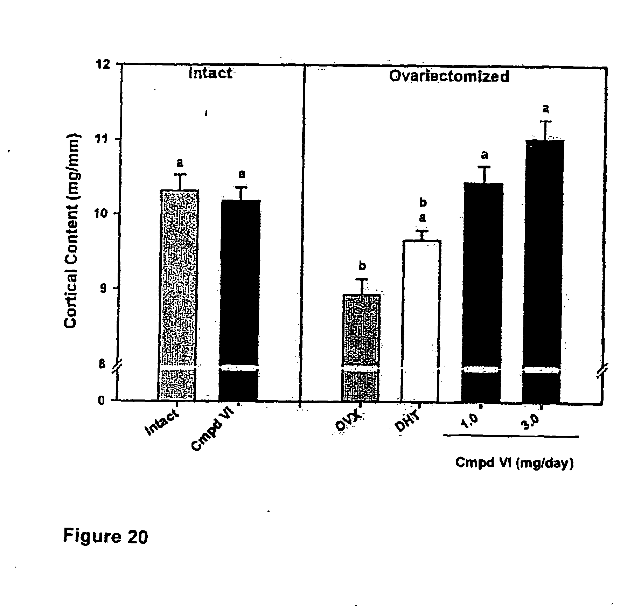

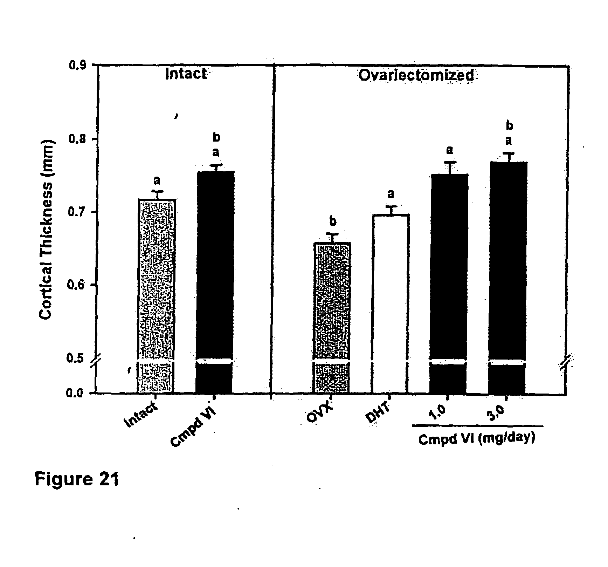

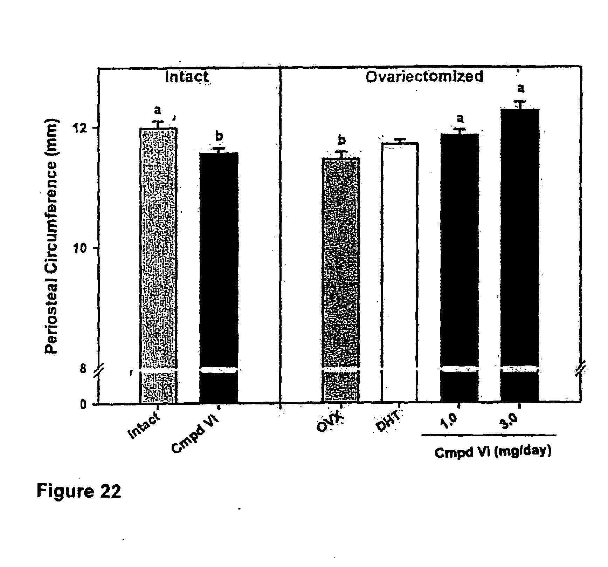

CC at the femoral mid-shaft was determined for the rats of Example 8. CC decreased from 10.3 to 8.9 mg/mm in OVX rats (Figure 20). The 1.0 mg/day and 3.0 mg/day doses of Compound VI partially (9.6 mg/mm) and fully (10.1 mg/mm) reversed the decline in CC, respectively. DHT fully restored CC to 9.9 mg/mm. CT decreased from 0.72 to 0.66 mm as a result of OVX; this decrease was significantly reversed in several of the Compound VI-treated groups (Figure 21). Similar to CC, PC decreased from 11.98 to 11.45 mm following OVX; the decreases were fully reversed in rats receiving 1 and 3 mg/day of Compound VI to 12.06 and 12.21 mm, respectively (Figure 22). DHT treatment resulted in a slight, non-statistically significant increase to 11.84 mm.

The results of this Example show that SARMS can reverse cortical bone loss resulting from osteoporosis.

### EXAMPLE 11

### COMPOUND VI REVERSES LOSS OF TRABECULAR BONE IN OSTEOPOROTIC SUBJECTS

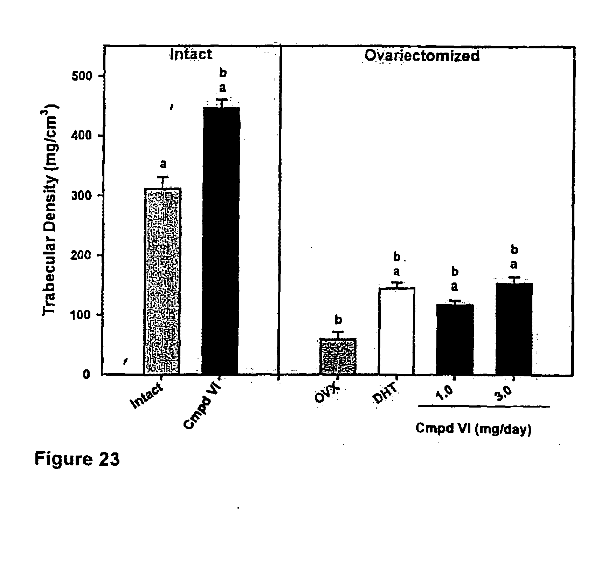

In addition, trabecular BMD was measured at the distal femur (Figure 23). Trabecular bone loss was evident in the distal femur following OVX. Both DHT and Compound VI partially restored trabecular BMD, showing that SARMS can partially reverse trabecular bone loss resulting from osteoporosis.

### EXAMPLE 12

### COMPOUND VI REVERSES BONE WEAKENING IN OSTEOPOROTIC SUBJECTS

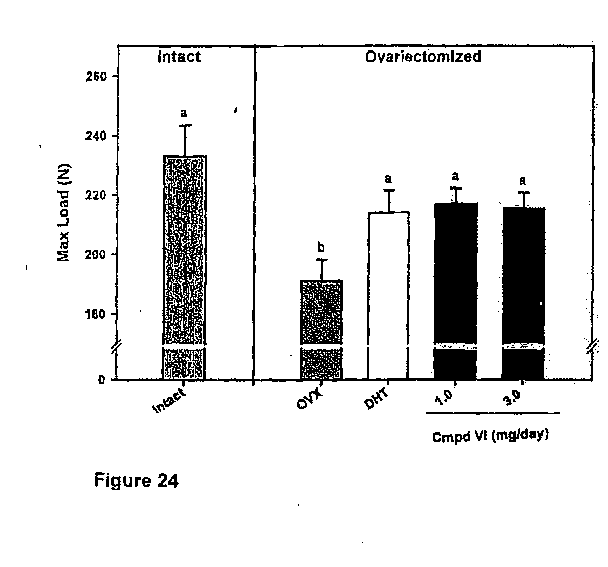

Biomechanical strength of the femurs of the rats of Example 8 was determined by three-point bending (Figure 24). OVX caused a reduction in the maximum load from 233 to 191 N. Treatment with 1.0 and 3.0 mg/day Compound VI increased the maximum load to 217 and 215 N, respectively, values not significantly different from the intact controls, showing that SARMS can reverse bone weakening resulting from osteoporosis. DHT treatment increased the maximum load to 214 N.

### EXAMPLE 13

### COMPOUND VI REVERSES INCREASED FM IN OSTEOPOROTIC SUBJECTS

Body weights of the rats of Example 8 increased by OVX from 308 to 336 g, and were further increased in a dose-dependent manner by Compound VI. (Figure 25). For example, groups treated with 0.1 and 3.0 mg/day of Compound VI averaged 350 and 381 g, respectively. Body weight of intact animals treated with Compound VI was the same as intact controls; while DHT treatment in intact animals resulted in an increase in body weight to 357 g.

Percent FM of the rats was assessed as well. FM in the OVX control group increased from 29% to 41 %. Compound VI treatment resulted in lower FM than the OVX control group in all dose groups, although the difference was not significant for some dose groups (Figure 26); a decrease was also seen with DHT treatment. Co-administration of bicalutamide with Compound VI partially abrogated the positive effects on FM seen with Compound VI treatment alone. Compound VI and DHT treatments of intact animals resulted in a 2% decrease and 8% increase in FM, respectively.

The findings of this Example show that (a) SARMS can reverse increased FM resulting from osteoporosis; and (b) SARMS can increase body mass in osteoporotic subj ects.

In summary, the findings of Examples 9-13 show that SARMS can reverse loss of BMD, loss of both cortical and trabecular bone, bone weakening, and increased FM in osteoporotic subjects. Since the drug was not added until after initiation of osteoporosis, the findings of these Examples assessed the anabolic activity, as opposed to the anti-resportive activity, of Compound VI. These findings corroborate the results of Examples 2-8, confirming the (a) osteo-anabolic activity and (b) protective activity against osteoporosis of SARMS.

### EXAMPLE 14

### COMPARISON OF COMPOUND VI WITH COMPOUNDS IX AND XI

Simultaneously with the studies described in Examples 1-13, the effect of Compound VI on skeletal growth and maintenance in the OVX model was compared to that of two structural analogs of Compound VI in which the para-nitro substituent of the A-ring was replaced with a para-cyano substituent and the para-acetamido substituent of the B-ring was replaced with a para-fluoro (Compound IX) or para-chloro substituent (Compound XI). Compounds were administered both immediately after OVX and 90 days subsequently. Rats were analyzed as described for Examples 2-13.

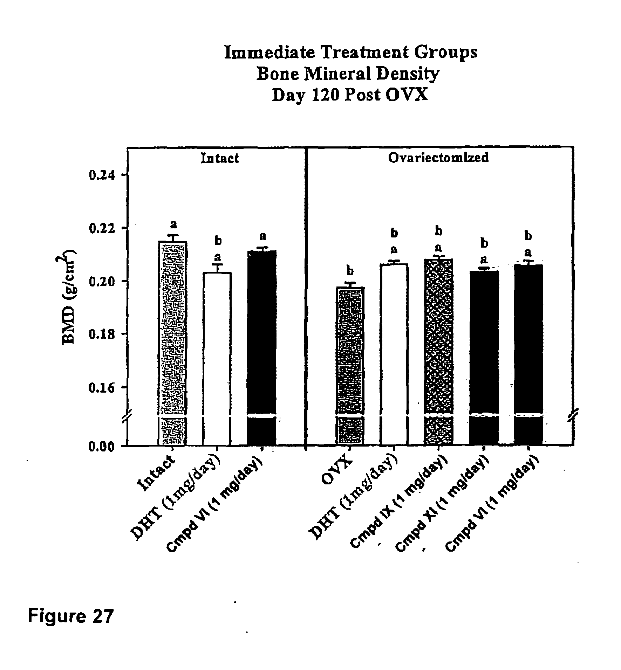

Figures 27-28 show the results for the immediate treatment groups at day 120. As expected, the BMD in OVX animals was significantly less than intact controls at day 120. Compounds VI, IX and XI all partially prevented BMD loss in the body as a whole (Figure 27).

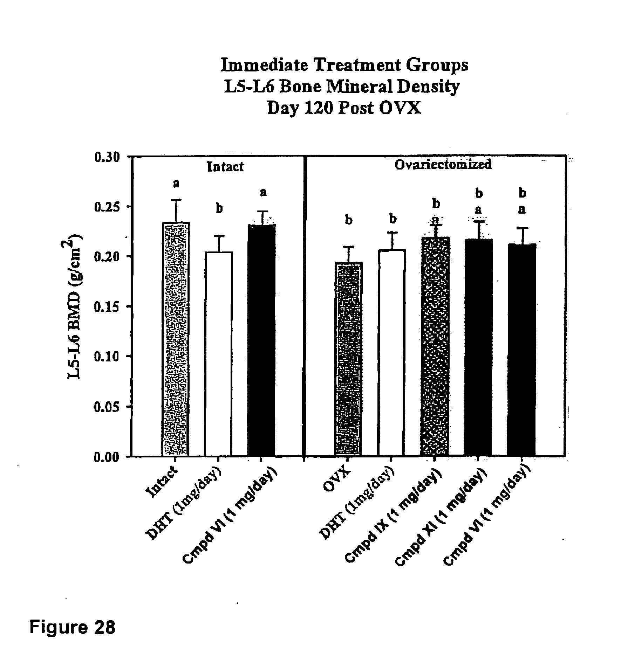

BMD of the L5-L6 vertebra was also assessed. OVX vehicle control animals lost a significant amount of BMD (Figure 28). 1 mg/day doses of Compounds VI, IX and XI, but no DHT, all partially prevented OVX-induced bone loss. Compound XI demonstrated the greatest effect on BMD in both whole body and L5-L6 vertebrae, although the effect was not statistically different from the other SARMs evaluated. DHT treatment in intact animals resulted in a significant decrease in BMD to a level similar to OVX controls, while BMD in intact animals receiving Compound VI was similar to intact controls.

These results show that, like Compound VI, Compounds IX and XI are potent SARMs that exhibit a bone protective effect and have application to treatment of muscle-wasting and osteoporosis.

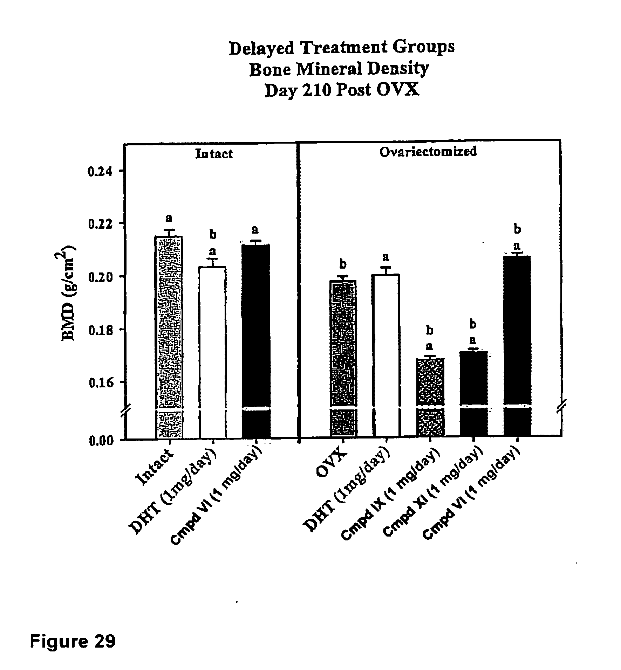

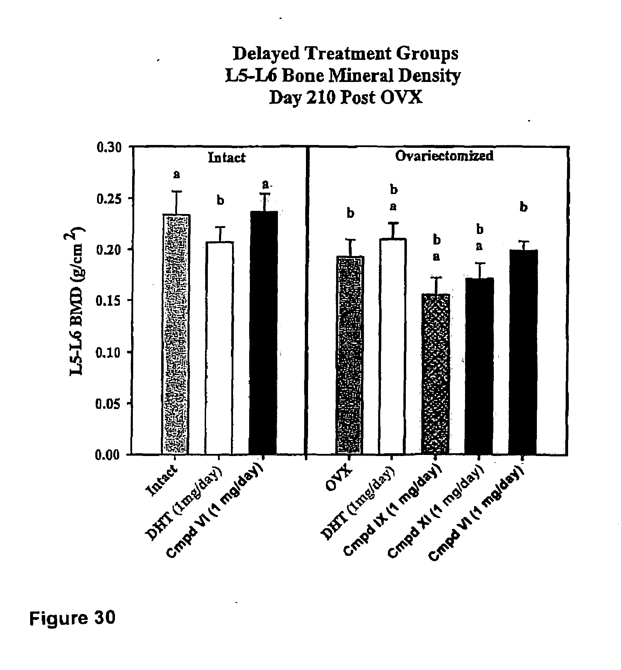

Figures 29-30 depict the BMD studies of the delayed treatment groups at day 210. OVX significantly decreased whole body BMD, which was partially prevented by Compound VI and DHT, but not Compound IX or XI (Figure 29). In the case of the L5-L6 vertebrae, DHT treatment also did not prevent loss of BMD (Figure 30). In intact animals, DHT, but not Compound VI, caused a significant decrease in BMD.

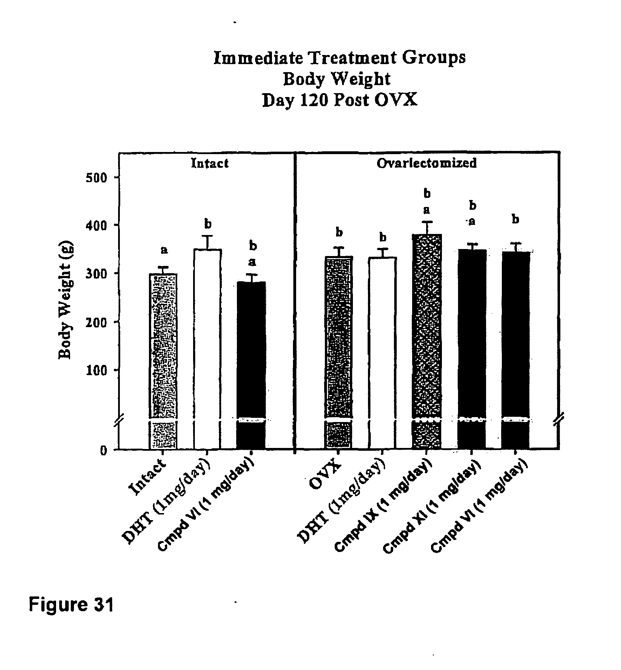

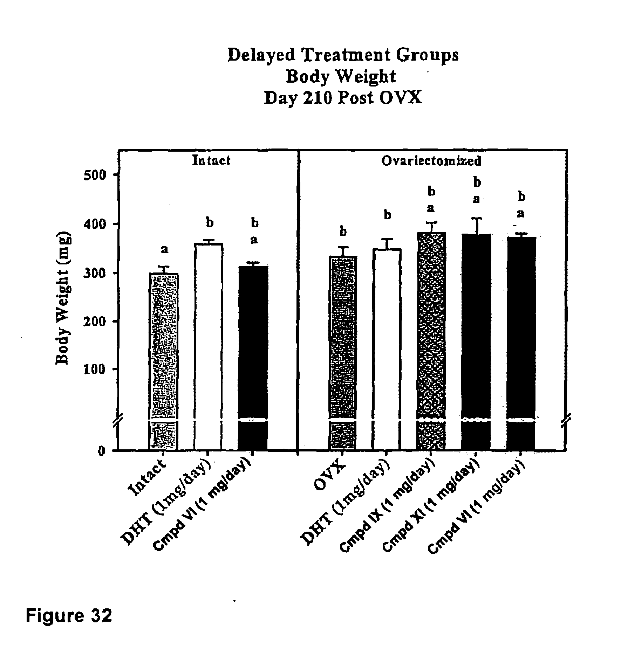

The average body weight for all immediate treatment groups was 262 ± 3 g (Mean ± S.D). All animals gained a significant amount of weight over the course of the study (Figure 31), which was further increased by OVX. Treatment with Compounds IX and IX further increased weight gain over intact or OVX controls. In intact animals, DHT, but not Compound VI, treatment resulted in further increases in body weight when compared with intact controls. Similar results were observed in the delayed treatment groups (Figure 32).

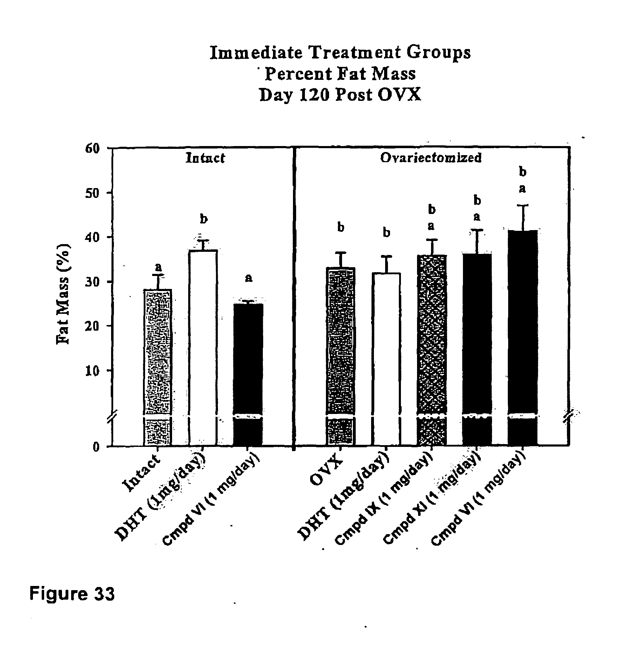

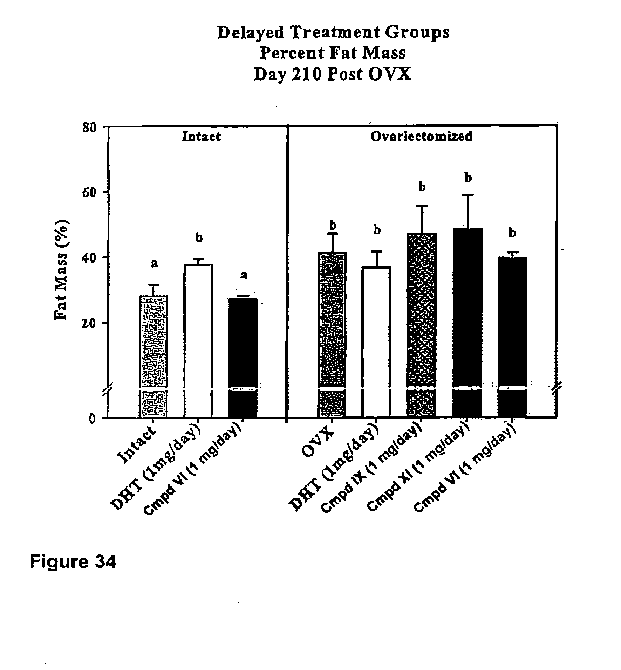

FM was increased by OVX, and further increased by treatment with Compound IX and XI (Figure 33). However, the increase was significantly less than that observed with Compound VI. DHT treatment in both intact and OVX animals increased FM to levels higher than intact controls but lower than OVX controls, respectively. Administration of Compound VI to intact rats decreased FM. In the delayed treatment groups, none of the treated OVX groups were significantly different from the OVX control group (Figure 34).

The results presented in this Example demonstrate that bone protective effects are not particular to Compound VI, but rather are also exhibited by other SARMs.

### EXAMPLE 15

### PHARMACO-KINETIC PROPERTIES OF COMPOUND VI

### Study design

Animals were randomized into seven groups, with five animals per group. Intravenous (i.v.) doses (0.5, 1, 10, and 30 mg kg⁻¹) were administered via the jugular vein catheter. Dosing solutions were prepared at an appropriate concentration to deliver the dose in a final volume of 0.2 to 0.3 ml. A 1 ml syringe graduated to 0.1 ml was used to volumetrically deliver the dose. After dose administration, the catheters were flushed with an aliquot (three times the volume of the administered dose) of sterile heparinized saline. Oral (p.o.) doses (1, 10, and 30 mg kg⁻¹) were introduced directly into the stomach via oral gavage in a volume of 0.2 to 0.3 ml. These doses were chosen to represent the range of Compound VI doses used during pre-clinical pharmacology, safety, and toxicology studies.

### Pharmacokinetics of Compound VI After I. V. Doses

Compound VI achieved average maximal plasma concentrations of 1.6, 2.3, 28, and 168 µg ml⁻¹ following i.v. doses of 0.5, 1, 10, and 30 mg kg⁻¹, respectively. The average steady state volume of distribution for Compound VI (0.45 L kg⁻¹) was slightly less than total body water (0.67 L kg⁻¹). CL remained relatively constant for the 0.5, 1 mg kg⁻¹, and 10 mg kg⁻¹ doses at 1.92, 2.12, and 1.52 ml min⁻¹ kg⁻¹, respectively. However, the CL of Compound VI was lower (1.00 ml min-1 kg⁻¹, p<0.05) at the 30 mg kg⁻¹ dose. Accordingly, the area under the plasma concentration time curve increased proportionally with dose up to the 10 mg kg⁻¹ dose. However, at an i.v. dose of 30 mg kg⁻¹, the AUC increased disproportionately to 29 mg min ml⁻¹. Urinary excretion data showed that less than 0.15% of the drug was excreted unchanged, indicating that renal elimination of Compound VI as unchanged drug was negligible. The T_{1/2} of Compound VI was 154, 182, 223, and 316 min after doses of 0.5, 1, 10, and 30 mg kg⁻¹, respectively. MRT increased from 222 and 240 min at the 0.5 and 1 mg kg⁻¹ doses to 305 and 423 min following the 10 and 30 mg kg⁻¹ doses, respectively, due to the decrease in clearance.

### Pharmacokinetics of Compound VI After P.O. Doses

Compound VI achieved average maximal plasma concentrations of 1.4, 11, and 20 µg ml⁻¹ following p.o. doses of 1, 10, and 30 mg kg⁻¹, respectively. The time to reach the maximal plasma concentration (Tₘₐₓ) was 48, 84, and 336 min for the 1, 10, and 30 mg kg⁻¹ doses, respectively. Compound VI was completely bioavailable for the 1 and 10 mg kg⁻¹ doses. However, following the 30 mg kg⁻¹ dose, the bioavailability of Compound VI decreased to 57%. The T_{1/2} of Compound VI was 203, 173, and 266 min after doses of 1, 10, and 30 mg kg⁻¹, respectively.

It will be appreciated by a person skilled in the art that the present invention is not limited by what has been particularly shown and described hereinabove. Rather, the scope of the invention is defined by the claims which follow:

## Claims

1. A compound represented by a structure of formula XVIII: 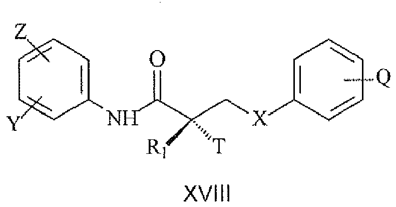 wherein
X is O;
T is OH, OR, -NHCOCH₃, or NHCOR;
Z is NO₂, CN, COOH, COR, NHCOR or CONHR;
Y is CF₃, F, I, Br, Cl, CN, C(R)₃ or Sn(R)₃;
Q is F, I, Br, Cl, CN, NHCOCH₃;
R is alkyl, haloalkyl, dihaloalkyl, trihaloalkyl, CH₂F, CHF₂, CF₃, CF₂CF₃, aryl, phenyl, F, Br, Cl, alkenyl or OH; and
R₁ is CH₃, CH₂F, CHF₂, CF₃, CH₂CH₃, or CF₂CF_{3;}
for use in (a) reducing fat mass; (b) increasing a lean mass, or a combination thereof in an osteoporotic subject.

2. A compound for use according to claim 1, represented by a structure: 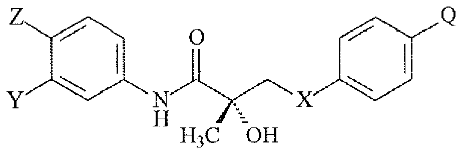 wherein
X is O;
Z is NO₂, CN, COOH, COR, NHCOR or CONHR;
Y is CF₃, F, Br, Cl, CN, C(R)₃ or Sn(R)₃;
Q is F, I, Br, Cl, CN, NHCOCH₃; and
R is alkyl, haloalkyl, dihaloalkyl, trihaloalkyl, CH₂F, CHF₂, CF₃, CF₂CF₃, aryl, phenyl, F, Br, Cl, alkenyl or OH.

3. A compound for use according to claim 1 represented by a structure of formula VI: 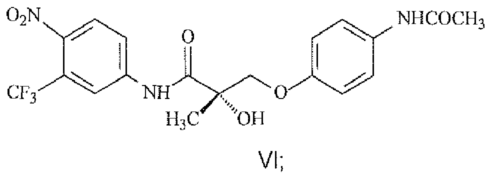 formula IX: 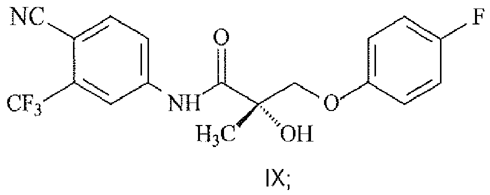 formula XI: 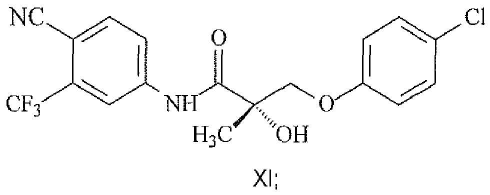 or formula XVI:

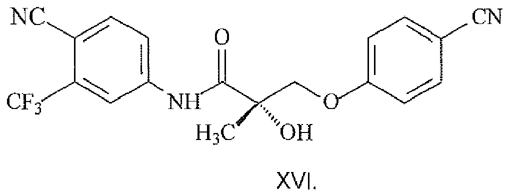

4. A compound for use according to any preceding claim, wherein said compound is a pharmaceutically acceptable salt, a hydrate, or a N-oxide, or any combination thereof.

5. A compound for use according to any preceding claim, wherein said use comprises administering a pharmaceutical composition comprising said compound and a pharmaceutically acceptable carrier.

6. A compound for use according to any preceding claim, wherein said subject has a hormonal imbalance, disorder, or disease.

7. A compound for use according to any preceding claim, wherein said subject has menopause.
